(19) Europäisches Patentamt
European Patent Office
Office européen des brevets

(11) Veröffentlichungsnummer : **0 541 486 A1**

# (12) EUROPÄISCHE PATENTANMELDUNG

(21) Anmeldenummer : **92810831.5**

(22) Anmeldetag : **28.10.92**

(51) Int. Cl.[5] : **C07H 15/26,** A61K 31/55

(30) Priorität : **07.11.91 CH 3244/91**
**09.09.92 CH 2828/92**

(43) Veröffentlichungstag der Anmeldung :
**12.05.93 Patentblatt 93/19**

(84) Benannte Vertragsstaaten :
**AT BE CH DE DK ES FR GB GR IE IT LI LU NL PT SE**

(71) Anmelder : **CIBA-GEIGY AG**
**Klybeckstrasse 141**
**CH-4002 Basel (CH)**

(72) Erfinder : **Wacker, Oskar**
**Löwenbergstrasse 60**
**CH-4059 Basel (CH)**
Erfinder : **Traxler, Peter, Dr.**
**Bündtenring 3**
**CH-4124 Schönenbuch (CH)**

(54) **Polycyclische Konjugate.**

(57) Beschrieben sind Staurosporinderivate der Formel I,

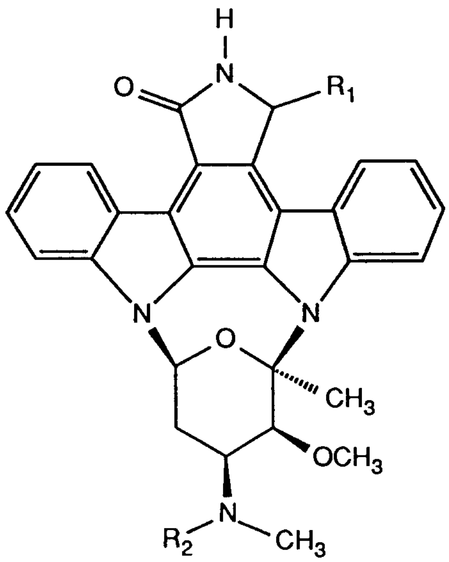

(I)

worin $R_1$ für Wasserstoff, Hydroxy, Niederalkoxy oder Oxo und $R_2$ für einen Rest der Formel II stehen,

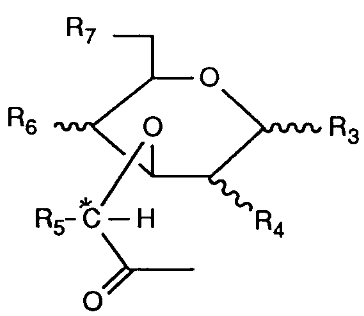

(II)

*(D) oder (L)-Konfiguration

worin sich die Konfiguration des Zuckerteils von D-Glucose, D-Galactose oder D-Mannose ableitet, und
$R_3$ Wasserstoff, Hydroxy, Niederalkanoyloxy, Niederalkoxy oder jeweils unsubstituiertes oder im Phenylteil durch Halogen, Hydroxy, Trifluormethyl, Niederalkyl oder Niederalkoxy substituiertes Benzyloxy, Benzoyloxy oder Phenyloxy,
$R_4$ Hydroxy, Niederalkanoyloxy, Benzoyloxy, Benzyloxy, Amino, Niederalkylamino, Diniederalkylamino,

EP 0 541 486 A1

Niederalkoxycarbonylamino, $C_2$-$C_{20}$-Alkanoylamino oder jeweils unsubstituiertes oder im Phenylteil durch Halogen, Hydroxy, Trifluormethyl, Niederalkyl oder Niederalkoxy substituiertes Benzoylamino, Benzyloxycarbonylamino oder Phenyloxycarbonylamino,
$R_5$ Wasserstoff oder Niederalkyl,
$R_6$ freies oder mit einer aliphatischen $C_2$-$C_{22}$-Carbonsäure verestertes Hydroxy, Niederalkoxycarbonyloxy, Niederalkylsulfonyloxy, freies oder mit einer aliphatischen $C_2$-$C_{22}$-Carbonsäure acyliertes Amino, Niederalkoxycarbonylamino, Azido oder jeweils unsubstituiertes oder im Phenylteil durch Halogen, Hydroxy, Trifluormethyl, Niederalkyl oder Niederalkoxy substituiertes Benzoyloxy, Benzyloxycarbonyloxy, Benzoylamino, Benzyloxycarbonylamino oder Phenylsulfonyloxy, und
$R_7$ freies oder mit einer aliphatischen $C_2$-$C_{22}$-Carbonsäure verestertes Hydroxy, Niederalkoxycarbonyloxy, Niederalkylsulfonyloxy, Azido, freies oder mit einer aliphatischen $C_2$-$C_{22}$-Carbonsäure acyliertes Amino, Niederalkylamino,
Diniederalkylamino, Niederalkoxycarbonylamino, Carbamoylamino oder jeweils unsubstituiertes oder im Phenylteil durch Halogen, Hydroxy, Trifluormethyl, Niederalkyl, Niederalkoxy oder Niederalkoxycarbonyl substituiertes Benzoyloxy,
Benzyloxycarbonyloxy, Phenylsulfonyloxy, Benzoylamino, Benzylamino oder Benzyloxycarbonylamino bedeuten.

Diese Verbindungen haben eine ausgeprägte und selektive Hemmwirkung auf das Enzym Proteinkinase C und besitzen unter anderem tumorhemmende Wirkung.

Die Erfindung betrifft Staurosporinderivate, die kovalent an Muraminsäurederivate konjugiert sind, Verfahren zu ihrer Herstellung, Arzneimittel, enthaltend diese Verbindungen, und deren Verwendung zur therapeutischen Behandlung von Warmblütern.

Staurosporin als Grundstoff der erfindungsgemässen Verbindungen wurde bereits im Jahre 1977 aus den Kulturen von Streptomyces staurosporeus AWAYA, TAKAHASHI and OMURA, sp. nov. AM 2282, vgl. S. Omura et al., J. Antibiot. 30, 275-281 (1977), isoliert und auf antimikrobielle Wirksamkeit geprüft. Es wurde dabei auch gefunden, dass die Verbindung gegen hefeartige Mikroorganismen und Pilze wirksam ist (MIC von etwa 3-25 mcg/ml). In letzer Zeit hat sich bei ausgedehntem Screening gezeigt, vgl. T. Tamaoki et al., Biochem. and Biophys. Research Commun. 135 (No. 2), 397-402 (1986), dass Staurosporin eine starke inhibierende Wirkung auf Proteinkinase C (aus Rattenhirn) ausübt.

Die von Phospholipiden und Calcium abhängige Proteinkinase C kommt innerhalb der Zelle in mehreren Formen vor und beteiligt sich an verschiedenen fundamentalen Vorgängen, wie Signalübertragung, Proliferation und Differenzierung, sowie auch Ausschüttung von Hormonen und Neurotransmittern. Die Aktivierung dieses Enzyms erfolgt bekanntlich entweder durch eine über Rezeptoren vermittelte Hydrolyse von Phospholipiden der Zellmembran oder durch eine direkte Interaktion mit gewissen Tumor-fördernden Wirkstoffen. Die Empfindlichkeit der Zelle gegenüber der rezeptorenvermittelten Signalübertragung kann durch die Abwandlung der Aktivität von Proteinkinase C (als Signalüberträger) wesentlich beeinflusst werden. Verbindungen, die fähig sind, die Aktivität der Proteinkinase C zu beeinflussen, können Anwendung als tumorhemmende, entzündungshemmende, immunomodulierende und antibakterielle Wirkstoffe finden und sogar als Mittel gegen Atherosklerose und Krankheiten des kardiovaskulären Systems und zentralen Nervensystems von Interesse sein.

Obwohl Staurosporin eine starke Hemmwirkung auf Proteinkinase C aufweist (siehe oben), hemmt es ebenso stark auch andere Proteinkinasen und hat deshalb nicht die Selektivität, welche für eine therapeutische Anwendung notwendig wäre.

Ziel der vorliegenden Erfindung war die Bereitstellung neuer Staurosporinderivate, die zwar die Hemmaktivität des Staurosporins gegenüber Proteinkinase C selektiv behalten, aber gegenüber anderen Proteinkinasen wesentlich weniger wirksam sind.

Es hat sich nun überraschenderweise gezeigt, dass durch kovalente Verknüpfung von Staurosporinderivaten an Muraminsäurederivate Verbindungen erhalten werden, die eine sehr starke Hemmwirkung auf Proteinkinase C entfalten, andere Proteinkinasen aber erst in einer um Grössenordnungen höheren Konzentration hemmen.

Die Erfindung betrifft insbesondere Staurosporinderivate der Formel I,

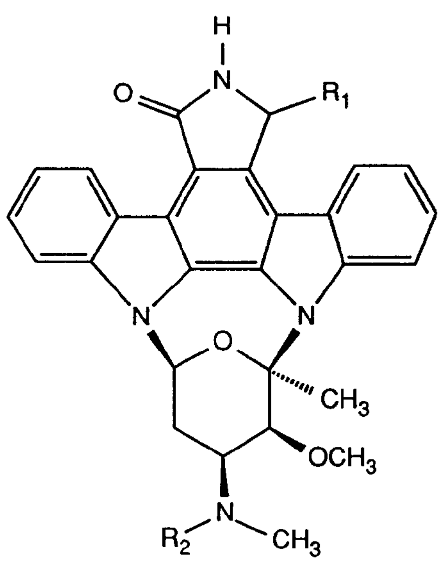

(I)

worin $R_1$ für Wasserstoff, Hydroxy, Niederalkoxy oder Oxo und $R_2$ für einen Rest der Formel II stehen,

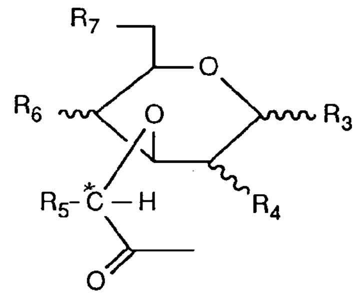

(II)

*(D) oder (L)-Konfiguration

worin sich die Konfiguration des Zuckerteils von D-Glucose, D-Galactose oder D-Mannose ableitet, und $R_3$ Wasserstoff, Hydroxy, Niederalkanoyloxy, Niederalkoxy oder jeweils unsubstituiertes oder im Phenylteil durch Halogen, Hydroxy, Trifluormethyl, Niederalkyl oder Niederalkoxy substituiertes Benzyloxy, Benzoyloxy oder Phenyloxy,

$R_4$ Hydroxy, Niederalkanoyloxy, Benzoyloxy, Benzyloxy, Amino, Niederalkylamino, Diniederalkylamino, Niederalkoxycarbonylamino, $C_2$-$C_{20}$-Alkanoylamino oder jeweils unsubstituiertes oder im Phenylteil durch Halogen, Hydroxy, Trifluormethyl, Niederalkyl oder Niederalkoxy substituiertes Benzoylamino, Benzyloxycarbonylamino oder Phenyloxycarbonylamino,

$R_5$ Wasserstoff oder Niederalkyl,

$R_6$ freies oder mit einer aliphatischen $C_2$-$C_{22}$-Carbonsäure verestertes Hydroxy, Niederalkoxycarbonyloxy, Niederalkylsulfonyloxy, freies oder mit einer aliphatischen $C_2$-$C_{22}$-Carbonsäure acyliertes Amino, Niederalokoxycarbonylamino, Azido oder jeweils unsubstituiertes oder im Phenylteil durch Halogen, Hydroxy, Trifluormethyl, Niederalkyl oder Niederalkoxy substituiertes Benzoyloxy, Benzyloxycarbonyloxy, Benzoylamino, Benzyloxycarbonylamino oder Phenylsulfonyloxy, und

$R_7$ freies oder mit einer aliphatischen $C_2$-$C_{22}$-Carbonsäure verestertes Hydroxy, Niederalkoxycarbonyloxy, Niederalkylsulfonyloxy, Azido, freies oder mit einer aliphatischen $C_2$-$C_{22}$-Carbonsäure acyliertes Amino, Niederalkylamino, Diniederalkylamino, Niederalkoxycarbonylamino, Carbamoylamino oder jeweils unsubstituiertes oder im Phenylteil durch Halogen, Hydroxy, Trifluormethyl, Niederalkyl, Niederalkoxy oder Niederalkoxycarbonyl substituiertes Benzoyloxy, Benzyloxycarbonyloxy, Phenylsulfonyloxy, Benzoylamino, Benzylamino oder Benzyloxycarbonylamino bedeuten, Säureadditionssalze dieser Verbindungen mit mindestens einer salzbildenden Gruppe, Verfahren zu ihrer Herstellung, Arzneimittel, enthaltend diese Verbindungen, und deren Verwendung zur therapeutischen Behandlung von Warmblütern.

Die vor- und nachstehend mit dem Präfix "Nieder-" bezeichneten Reste enthalten bis und mit 7, vorzugsweise bis und mit 4 C-Atome.

Niederalkoxy $R_1$ ist vorzugsweise Methoxy, Ethoxy, Propoxy oder Butoxy. In sterischer Hinsicht kann sich der Rest $R_1$ in α- oder β-Position befinden.

Reste der Formel II, worin sich die Konfiguration des Zuckerteils von D-Glucose ableitet, sind bevorzugt. Diese Reste entsprechen der Formel III.

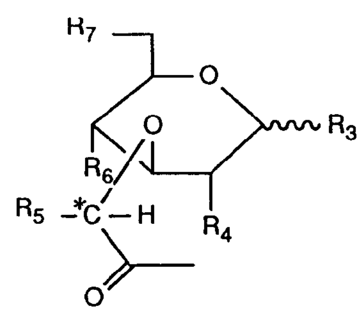

(III)

*(D)- oder (L)-Konfiguration

In den Resten der Formeln II und III ist die Konfiguration am C-$R_5$-Atom im Falle asymmetrischer Substitution, d.h. wenn $R_5$ von Wasserstoff verschieden ist, L oder vorzugsweise D, und die Gruppe $R_3$ ist im Falle $R_3$ = OH α- und β-ständig und im Falle $R_3 \neq$ OH vorzugsweise α-ständig.

Die Verbindungen der Formel I können auch als Derivate der Muraminsäure aufgefasst werden, welche Formel IV hat.

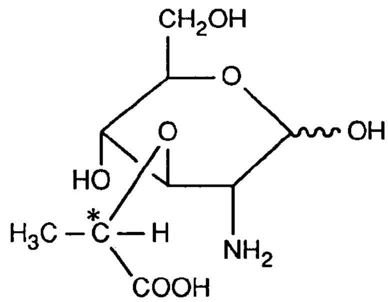

(IV)

* (D)-Konfiguration

Der entsprechende Acylrest wird "Muramyl" genannt. (L)-Muraminsäure hat die unnatürliche (L)-Konfiguration am $*\underline{C}$-$CH_3$. Als Desmethylmuraminsäure wird die Verbindung der Formel IV bezeichnet, worin die Methylgruppe durch Wasserstoff ersetzt ist. Als Homomuraminsäure wird die Verbindung der Formel IV bezeichnet, worin die Methylgruppe durch Ethyl ersetzt ist.

Wie ersichtlich ist, sind die Verbindungen der Formel I Amide von Muraminsäurederivaten oder ihren Diastereomeren.

Niederalkanoyloxy $R_3$ ist vorzugsweise Acetoxy oder Propionyloxy. Niederalkoxy $R_3$ ist vorzugsweise Methoxy oder Ethoxy.

Benzyloxy, Benzoyloxy oder Phenyloxy $R_3$ ist vorzugsweise unsubstituiert. Diese Reste können jedoch auch durch einen oder mehrere, in der Regel aber nicht mehr als drei gleiche oder verschiedene der obengenannten Phenylsubstituenten substituiert sein.

Halogen als Phenylsubstituent ist vorzugsweise Fluor, Chlor oder Brom, daneben aber auch Iod. Niederalkyl als Substituent eines Phenylteiles ist vorzugsweise Methyl. Niederalkoxy als Phenylsubstituent ist vorzugsweise Methoxy.

Niederalkylamino $R_4$ ist vorzugsweise Methylamino oder Ethylamino. Diniederalkylamino $R_4$ ist vorzugsweise Dimethylamino oder Diethylamino. Niederalkoxycarbonylamino $R_4$ ist vorzugsweise tert. Butyloxycarbonylamino. $C_2$-$C_{20}$-Alkanoylamino $R_4$ ist vorzugsweise Acetylamino oder Propionylamino. Benzoylamino, Benzyloxycarbonylamino oder Phenyloxycarbonylamino $R_4$ ist vorzugsweise unsubstituiert. Diese Reste können jedoch auch durch einen oder mehrere, in der Regel aber nicht mehr als drei gleiche oder verschiedene der obengenannten Phenylsubsrituenten substituiert sein. Durch Niederalkyl substituiertes Phenylsulfonyloxy ist vorzugsweise p-Toluolsulfonyloxy.

Niederalkyl $R_5$ ist vorzugsweise Methyl, ferner Ethyl.

Eine aliphatische $C_2$-$C_{22}$-Carbonsäure als Veresterungskomponente von verestertem Hydroxy oder acyliertem Amino $R_6$ oder $R_7$ ist insbesondere eine unsubstituierte aliphatische $C_2$-$C_{22}$-Carbonsäure, insbesondere eine $C_2$-$C_{22}$-Alkansäure oder eine $C_2$-$C_{22}$-Alkensäure, hauptsächlich eine unverzweigte $C_2$-$C_{22}$-Alkansäure oder eine unverzweigte $C_2$-$C_{22}$-Alkensäure, vorzugsweise Essigsäure oder Propionsäure, daneben, insbesondere im Falle des Restes $R_7$, aber auch z.B. Laurinsäure, Palmitinsäure, Stearinsäure oder Oelsäure.

Niederalkoxycarbonyloxy $R_6$ oder $R_7$ ist beispielsweise tert. Butyloxycarbonyloxy. Niederalkylsulfonyloxy $R_6$ oder $R_7$ ist vorzugsweise Methylsulfonyloxy.

Benzoyloxy, Benzyloxycarbonyloxy, Benzoylamino, Benzyloxycarbonylamino oder Phenylsulfonyloxy $R_6$ oder $R_7$ oder Benzylamino $R_7$ ist vorzugsweise unsubstituiert. Diese Reste können jedoch auch durch einen oder mehrere, in der Regel aber nicht mehr als drei gleiche oder verschiedene der obengenannten Phenylsubstituenten substituiert sein.

Die Verbindungen der Formel I mit mindestens einer basischen Gruppe, z.B. einer freien Aminogruppe als Rest $R_4$ oder $R_7$, können Säureadditionssalze bilden, z.B. mit anorganischen Säuren, wie Salzsäure, Schwefelsäure oder einer Phosphorsäure, oder mit geeigneten organischen Carbon- oder Sulfonsäuren, z.B. aliphatischen Mono- oder Dicarbonsäuren, wie Trifluoressigsäure, Essigsäure, Propionsäure, Glykolsäure, Bernsteinsäure, Maleinsäure, Fumarsäure, Hydroxymaleinsäure, Aepfelsäure, Weinsäure, Zitronensäure, Oxalsäure oder Aminosäuren, wie Arginin oder Lysin, aromatischen Carbonsäuren, wie Benzoesäure, 2-Phenoxy-benzoesäure, 2-Acetoxy-benzoesäure, Salicylsäure, 4-Aminosalicylsäure, aromatisch-aliphatischen Carbonsäuren, wie Mandelsäure oder Zimtsäure, heteroaromatischen Carbonsäuren, wie Nicotinsäure oder Isonicotinsäure, aliphatischen Sulfonsäuren, wie Methan-, Ethan- oder 2-Hydroxy-ethan-sulfonsäure, oder aromarischen Sulfonsäuren, z.B. Benzol-, p-Toluol- oder Naphthalin-2-sulfonsäure. Bei Anwesenheit von mehreren basischen Gruppen können Mono- oder Polysäureadditionssalze gebildet werden.

Zur Isolierung oder Reinigung sowie bei den als Zwischenprodukt weiterverwendeten Verbindungen können auch pharmazeurisch ungeeignete Salze Verwendung finden. Zur therapeutischen Anwendung gelangen jedoch nur die pharmazeutisch verwendbaren, nicht-toxischen Salze, die deshalb bevorzugt werden.

Infolge der engen Beziehung zwischen den neuen Verbindungen in freier Form und in Form ihrer Salze, inkl. auch solcher Säureadditionssalze, die als Zwischenprodukte, z.B. bei der Reinigung der neuen Verbindungen oder zu ihrer Identifikation verwendet werden können, sind vorausgehend und nachfolgend unter den freien Verbindungen sinn- und zweckgemäss gegebenenfalls auch die entsprechenden Salze zu verstehen.

Die Verbindungen der Formel I besitzen wertvolle pharmakologische Eigenschaften, z.B. hemmen sie selektiv das Enzym Proteinkinase C. Zur Bestimmung der Proteinkinase-C-Hemmwirkung verwendet man Proteinkinase C aus Schweinehirn, welche gemäss der von T. Uchida und C.R. Filburn in J. Biol. Chem. 259, 12311-4 (1984) beschriebenen Verfahrensweise gereinigt wird. Die Bestimmung der Proteinkinase-C-Hemmwirkung der Verbindungen der Formel I erfolgt nach der Methodik von D. Fabbro et al., Arch. Biochem. Biophys. 239, 102-111 (1985). In diesem Test hemmen die Verbindungen der Formel I die Proteinkinase C bereits bei einer Konzentration $IC_{50}$ zwischen etwa 0,001 und 0,05 $\mu$Mol/Liter. Demgegenüber hemmen die Verbindungen der Formel I andere Enzyme, z.B. Proteinkinase A, Protein-Phosphorylase-Kinase und Protein-Tyrosin-Kinase, erst bei einer weitaus, z.B 100fach höheren Konzentration. Dies zeigt die Selektivität der Verbindungen der Formel I.

Wie bereits aufgrund der obengeschilderten Hemmwirkung auf Proteinkinase C erwartet werden kann, weisen die Verbindungen der Formel I antiproliferative Eigenschaften auf, die sich in folgendem anderen Versuch direkt demonstrieren lassen: Dabei wird die Hemmwirkung der Verbindungen der Formel I auf das Wachstum von menschlichen T24 Blasenkarzinomzellen bestimmt. Diese Zellen werden in "Eagle's minimal essential medium", dem 5 % (V/V) fötales Kälberserum zugesetzt sind, in einem befeuchteten Inkubator bei 37°C und 5 Volumenprozent $CO_2$ in der Luft inkubiert. Die Karzinomzellen (1000-1500) werden in 96-Loch-Mikrotiterplatten überimpft und über Nacht unter den obengenannten Bedingungen inkubiert. Die Testsubstanz wird in seriellen Verdünnungen am Tag 1 hinzugefügt. Die Platten werden unter den obengenannten Bedingungen 5 Tage lang inkubiert. Während dieser Zeitspanne durchlaufen die Kontrollkulturen mindestens 4 Zellteilungen. Nach der Inkubation werden die Zellen mit 3,3%iger (G/V) wässriger Glutaraldehydlösung fixiert, mit Wasser gewaschen und mit 0,05 %iger (Gewicht/Volumen) wässriger Methylenblaulösung gefärbt. Nach dem Waschen wird der Farbstoff mit 3%iger (G/V) wässriger Salzsäure eluiert. Danach wird die optische Dichte (OD) pro Loch, welche der Zellanzahl direkt proportinal ist, mit einem Photometer (Titertek multiskan) bei 665 nm gemessen. Die $IC_{50}$-Werte werden mit einem Computersystem unter Verwendung der Formel

$$\frac{OD_{665}\text{ (Test) minus }OD_{665}\text{ (Anfang)}}{OD_{665}\text{ (Kontrolle) minus }OD_{665}\text{ (Anfang)}} \times 100$$

errechnet. Die $IC_{50}$-Werte sind als diejenige Wirkstoffkonzentration definiert, bei der die Anzahl der Zellen pro Loch am Ende der Inkubationszeit nur 50 % der Zellanzahl in den Kontrollkulturen beträgt. Die so ermittelten $IC_{50}$-Werte liegen für die Verbindungen der Formel I zwischen etwa 0,01 und 0,4 $\mu$Mol/Liter.

Aufgrund der beschriebenenen Eigenschaften können die Verbindungen der Formel I insbesondere als tumorhemmende Wirkstoffe verwendet werden, z.B zur Therapie von Tumoren der Blase. Ausserdem kommen sie für die oben für Proteinkinase C Modulatoren genannten weiteren Anwendungen in Betracht und können insbesondere zur Behandlung von Krankheiten verwendet werden, die auf eine Hemmung der Proteinkinase C ansprechen.

Bevorzugt sind Verbindungen der Formel I, worin $R_3$ Hydroxy, Niederalkanoyloxy, Niederalkoxy oder jeweils unsubstituiertes oder im Phenylteil durch Halogen, Hydroxy, Trifluormethyl, Niederalkyl oder Niederalkoxy substituiertes Benzyloxy, Benzoyloxy oder Phenyloxy und $R_4$ Amino, Niederalkylamino, Diniederalokylamino, Niederalkoxycarbonylamino, $C_2$-$C_{20}$-Alkanoylamino oder jeweils unsubstituiertes oder im Phenylteil durch Halogen, Hydroxy, Trifluormethyl, Niederalkyl oder Niederalkoxy substituiertes Benzoylamino, Benzyloxycarbonylamino oder Phenyloxycarbonylamino bedeuten, und/oder worin

$R_6$ freies oder mit einer unsubstituierten aliphatischen $C_2$-$C_{22}$-Carbonsäure, vorzugsweise einer unverzweigten $C_2$-$C_{22}$-Alkansäure oder einer unverzweigten $C_2$-$C_{22}$-Alkensäure, verestertes Hydroxy, Niederalkoxycarbonyloxy, Niederalkylsulfonyloxy, freies oder mit einer unsubstituierten aliphatischen $C_2$-$C_{22}$-Carbonsäure, vorzugsweise einer unverzweigten $C_2$-$C_{22}$-Alkansäure oder einer unverzweigten $C_2$-$C_{22}$-Alkensäure, acyliertes Amino, Niederalkoxycarbonylamino, Azido oder jeweils unsubstituiertes oder im Phenylteil durch Halogen, Hydroxy, Trifluormethyl, Niederalkyl oder Niederalkoxy substituiertes Benzoyloxy, Benzyloxycarbonyloxy, Benzoylamino, Benzyloxycarbonylamino oder Phenylsulfonyloxy, und

$R_7$ freies oder mit einer unsubstituierten aliphatischen $C_2$-$C_{22}$-Carbonsäure, vorzugsweise einer unverzweigten $C_2$-$C_{22}$-Alkansäure oder einer unverzweigten $C_2$-$C_{22}$-Alkensäure, verestertes Hydroxy, Niederalkoxycarbonyloxy, Niederalkylsulfonyloxy, Azido, freies oder mit einer unsubstituierten aliphatischen $C_2$-$C_{22}$-Carbonsäure, vorzugsweise einer unverzweigten $C_2$-$C_{22}$-Alkansäure oder einer unverzweigten $C_2$-$C_{22}$-Alkensäure, acyliertes Amino, Niederalkylamino, Diniederalkylamino, Niederalkoxycarbonylamino, Carbamoylamino oder jeweils unsubstituiertes oder im Phenylteil durch Halogen, Hydroxy, Trifluormethyl, Niederalkyl, Niederalkoxy oder Niederalkoxycarbonyl substituiertes Benzoyloxy, Benzyloxycarbonyloxy, Phenylsulfonyloxy, Benzoylamino, Benzylamino oder Benzyloxycarbonylamino bedeuten, insbesondere die vorstehend genannten Verbindungen der Formel I, worin $R_1$ Wasserstoff bedeutet, und Säureadditionssalze von solchen Verbindungen mit mindestens einer salzbildenden Gruppe.

Bevorzugt sind insbesondere Verbindungen der Formel I, worin $R_1$ Wasserstoff und $R_2$ einen Rest der Formel II bedeuten, worin $R_3$ Hydroxy, Benzyloxy, welches vorzugsweise $\alpha$-ständig ist, oder Niederalkoxy, welches vorzugsweise $\alpha$-ständig ist, $R_4$ Niederalkanoylamino, $R_5$ Niederalkyl oder Wasserstoff, $R_6$ Hydroxy und $R_7$ Hydroxy, Niederalkylsulfonyloxy, Azido oder Amino bedeuten, und Säureadditionssalze von solchen Verbindungen mit mindestens einer salzbildenden Gruppe.

Besonders bevorzugt sind Verbindungen der Formel I, worin sich die Konfiguration des Zuckerteils von D-Glucose ableitet und/oder die am C∗-Atom die (D)-Konfiguration aufweisen, und Säureadditionssalze von solchen Verbindungen mit mindestens einer salzbildenden Gruppe.

Stark bevorzugt sind die Verbindungen der Formel I, worin sich die Konfiguration des Zuckerteils von D-Glucose ableitet, $R_1$ Wasserstoff und $R_2$ einen Rest der Formel II bedeuten, worin $R_3$ Wasserstoff, Hydroxy, Benzyloxy, Niederalkoxy oder Niederalkanoyloxy, $R_4$ Niederalkanoylamino, vorzugsweise $C_1$-$C_4$-Alkanoylamino, $R_5$ Niederalkyl, vorzugsweise $C_1$-$C_4$-Alkyl, oder Wasserstoff, $R_6$ Hydroxy oder Niederalkanoyloxy und $R_7$ Hydroxy, Niederalkylsulfonyloxy, vorzugsweise Methylsulfonyloxy, Azido, Amino oder Alkanoyloxy mit bis zu 24 C-Atomen bedeuten, sowie entsprechende Verbindungen, worin $R_7$ p-Toluolsulfonyloxy bedeutet, und Säureaddirionssalze von solchen Verbindungen mit mindestens einer salzbildenden Gruppe.

Ganz besonders bevorzugt sind die Verbindungen der Formel I, worin sich die Konfiguration des Zuckerteils von D-Glucose ableitet, $R_1$ Wasserstoff und $R_2$ einen Rest der Formel II bedeuten, worin $R_3$ Hydroxy, Benzyloxy oder Niederalkoxy, $R_4$ Niederalkanoylamino, $R_5$ Niederalkyl oder Wasserstoff, $R_6$ Hydroxy und $R_7$ Hydroxy, Niederalkylsulfonyloxy, Azido oder Amino bedeuten, und Säureadditionssalze von solchen Verbindungen mit mindestens einer salzbildenden Gruppe.

Am meisten bevorzugt sind die in den Beispielen beschriebenen Verbindungen der Formel I und Säureadditionssalze von solchen Verbindungen mit mindestens einer salzbildenden Gruppe.

Die Verbindungen der Formel I und die Säureadditionssalze von solchen Verbindungen mit mindestens einer salzbildenden Gruppe werden nach an sich bekannten Verfahren hergestellt. Das erfindungsgemässe Verfahren ist dadurch gekennzeichnet, dass man ein Amin der Formel V,

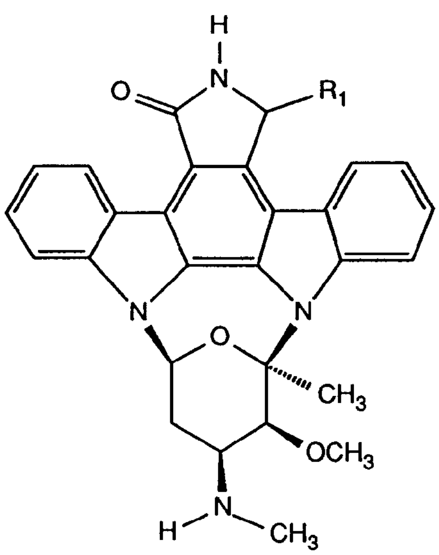

(V)

worin $R_1$ die obengenannte Bedeutung hat mit der Massgabe, dass eine durch $R_1$ repräsentierte Hydroxygruppe erforderlichenfalls durch eine leicht abspaltbare Hydroxyschutzgruppe geschützt ist, mit einer Carbonsäure der Formel VI (Pyranoseform),

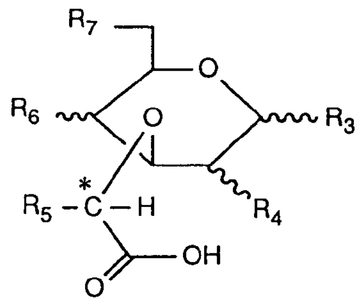

(VI)

*(D) oder (L)-Konfiguration

worin die Substituenten die obengenannten Bedeutungen haben mit der Massgabe, dass in einer Verbindung der Formel VI vorhandene freie funktionelle Gruppen mit Ausnahme der an der Reaktion teilnehmenden Carboxylgruppe erforderlichenfalls durch leicht abspaltbare Schutzgruppen geschützt sind, oder einer entsprechenden Carbonsäure in der Furanoseform, oder einem reaktionsfähigen Carbonsäurederivat davon acyliert, und Schutzgruppen, welche im gewünschten Endprodukt der Formel I nicht vorhanden sind, abspaltet und, wenn erwünscht, eine erhaltene Verbindung der Formel I mit mindestens einer salzbildenden Gruppe in eine andere Verbindung der Formel I überführt oder eine erhaltene Verbindung der Formel I in ihr Säureadditionssalz überführt, oder ein erhaltenes Säureadditionssalz einer Verbindung der Formel I in die freie Verbindung umwandelt, und/oder ein erhaltenes Isomerengemisch auftrennt.

Die Durchführung des obengenannten Verfahrens wird im folgenden näher erläutert:

Eine der Carbonsäure der Formel VI entsprechende Carbonsäure in der Furanoseform ist z.B. eine Verbindung der Formel VIII,

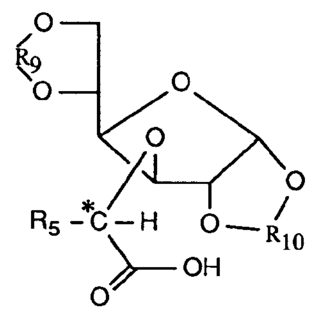

(VIII)

*(D) oder (L)-Konfiguration

worin $R_5$ die obengenannte Bedeutung hat und $R_9$ und $R_{10}$ je bivalente Hydroxyschutzgruppen bedeuten, oder eine Verbindung der Formel IX,

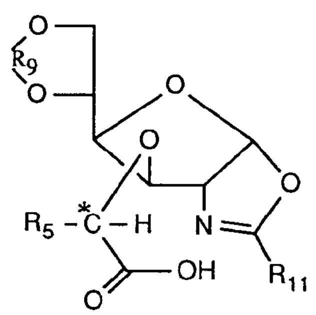

(IX)

*(D) oder (L)-Konfiguration

worin $R_5$ und $R_9$ die obengenannten Bedeutungen haben und $R_{11}$ $C_1$-$C_{19}$-Alkyl oder jeweils unsubstituiertes oder im Phenylteil durch Halogen, geschütztes Hydroxy, Trifluormethyl, Niederalkyl oder Niederalkoxy substituiertes Phenyl bedeutet. Geeignete bivalente Hydroxyschutzgruppen $R_9$ und $R_{10}$ sind z.B. unsubstituierte oder substituierte Alkyliden-oder Cycloalkylidenreste, vorzugsweise unsubstituierte oder substituierte Niederalkylidenreste, z.B. Ethyliden, Propyliden oder insbesondere Isopropyliden oder Benzyliden, welches z.B. in para-Stellung des Phenylteils substituiert sein kann, oder Cycloalkylidenreste, z.B. Cyclopentyliden oder Cyclohexyliden.

Allgemeines:

Die Endstoffe der Formel I können Substituenten, z.B. $R_3$ = Benzyloxy, enthalten, die auch als Schutzgruppen in Ausgangsstoffen zur Herstellung anderer Endstoffe der Formel I verwendet werden können. Als "Schutzgruppe" wird daher im Rahmen dieses Textes, wenn aus dem Zusammenhang nicht anders ersichtlich, nur eine solche leicht abspaltbare Gruppe bezeichnet, die nicht Bestandteil des jeweils gewünschten Endstoffes der Formel I ist.

Funktionelle Gruppen in einer Verbindung der Formel VI, die am besten durch leicht abspaltbare Schutzgruppen geschützt werden, sind in erster Linie freie Aminogruppen und daneben auch freie Hydroxygruppen. Schutzgruppen, ihre Einführung und Abspaltung sind beispielsweise beschrieben in "Protective Groups in Organic Chemistry", Plenum Press, London, New York 1973, und in "Methoden der organischen Chemie", Houben-Weyl, 4. Auflage, Bd. 15/1, Georg-Thieme-Verlag, Stuttgart 1974 sowie in Theodora W. Greene, "Protective Groups in Organic Synthesis, John Wiley & Sons, New York 1981. Charakteristisch für Schutzgruppen ist, dass sie leicht, d.h. ohne dass unerwünschte Nebenreaktionen stattfinden, z.B. solvolytisch, reduktiv, photolytisch oder auch unter physiologischen Bedingungen abspaltbar sind.

Hydroxyschutzgruppen sind z.B. Acylreste, wie gegebenenfalls, z.B. durch Halogen, substituiertes Niederalkanoyl, wie 2,2-Dichloracetyl, oder Acylreste von Kohlensäurehalbestern, insbesondere tert.-Butyloxycarbonyl, gegebenenfalls substituiertes Benzyloxycarbonyl, z.B. 4-Nitrobenzyloxycarbonyl, oder Diphenylmethoxycarbonyl, oder 2-Halogen-niederalkoxycarbonyl, wie 2,2,2-Trichlorethoxycarbonyl, ferner Trityl oder Formyl, oder organische Silyl- oder Stannylreste, ferner leicht abspaltbare verethernde Gruppen, wie tert.-Niederalkyl, z.B. tert.-Butyl, 2-oxa-oder 2-thia-aliphatische oder -cycloaliphatische Kohlenwasserstoffreste, in erster Linie 1-Niederalkoxyniederalkyl oder 1-Niederalkylthioniederalkyl, z.B. Methoxymethyl, 1-Methoxyethyl, 1-Ethoxyethyl, Methylthiomethyl, 1-Methylthioethyl oder 1-Ethylthioethyl, oder 2-oxa- oder 2-Thiacycloalkyl mit 5-6 Ringatomen, z.B. Tetrahydrofuryl oder 2-Tetrahydropyranyl oder entsprechende Thiaanaloge, sowie gegebenenfalls substituiertes 1-Phenylniederalkyl, wie gegebenenfalls substituiertes Benzyl oder Diphenylmethyl, wobei als Substituenten der Phenylreste z.B. Halogen, wie Chlor, Niederalkoxy, wie Methoxy und/oder Nitro in Frage kommen.

Eine bevorzugte Schutzgruppe für die Hydroxygruppe $R_3$ ($R_3$ = OH) in 1-Stellung des Zuckerteils der Verbindungen der Formel I ist Benzyl.

Eine geschützte Aminogruppe kann z.B. in Form einer leicht spaltbaren Acylamino-, Arylmethylamino-, verätherten Mercaptoamino-, 2-Acyl-nieder-alk-1-en-yl-amino-, Silyl-oder Stannylaminogruppe oder als Azidogruppe vorliegen.

In einer entsprechenden Acylaminogruppe ist Acyl beispielsweise der Acylrest einer organischen Carbonsäure mit z.B. bis zu 18 Kohlenstoffatomen, insbesondere einer gegebenenfalls, z.B. durch Halogen oder Aryl, substituierten Alkancarbonsäure oder gegebenenfalls, z.B. durch Halogen, Niederalkoxy oder Nitro, substituierten Benzoesäure, oder eines Kohlensäurehalbesters. Solche Acylgruppen sind beispielsweise Niederalkanoyl, wie Formyl, Acetyl oder Propionyl, Halogenniederalkanoyl, wie 2-Halogenacetyl, insbesondere 2-Chlor-, 2-Brom-, 2-Iod-, 2,2,2-Trifluor- oder 2,2,2-Trichloracetyl, gegebenenfalls, z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung des Niederalkylrestes verzweigtes oder in 1- oder 2-Stellung geeignet substituiertes Niederalkoxycarbonyl, insbesondere tert.-Niederalkoxycarbonyl, z.B. tert.- Butyloxycarbonyl, Arylmethoxycarbonyl mit einem oder zwei Arylresten, die vorzugsweise gegebenenfalls, z.B. durch Niederalkyl, insbesondere tert.-Niederalkyl, wie tert.Butyl, Niederalkoxy, wie Methoxy, Hydroxy, Halogen, z.B. Chlor, und/oder Nitro, mono- oder polysubstituiertes Phenyl darstellen, wie gegebenenfalls substituiertes Benzyloxycarbonyl, z.B. 4-Nitro-benzyloxycarbonyl, oder substituiertes Diphenylmethoxycarbonyl, z.B. Benzhydryloxycarbonyl oder Di-(4-methoxyphenyl)-methoxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogen-niederalkoxycarbonyl, z.B. 2,2,2-Trichlorethoxycarbonyl, 2-Bromethoxycarbonyl oder 2-Iodethoxycarbonyl, oder 2-(trisubstituiertes Silyl)-ethoxycarbonyl, worin die Substituenten unabhängig voneinander je einen gegebenenfalls substituierten, z.B. durch Niederalkyl, Niederalkoxy, Aryl, Halogen oder Nitro substituierten, aliphatischen, araliphatischen, cycloaliphatischen oder aromatischen Kohlenwasserstoffrest mit bis zu 15 C-Atomen, wie entsprechendes, gegebenenfalls substituiertes Niederalkyl, Phenylniederalkyl, Cycloalokyl oder Phenyl, bedeuten, z.B. 2-Triniederalkylsilylethoxycarbonyl, wie 2-Trimethylsilylethoxycarbonyl oder 2-(Di-n-butyl-methyl-silyl)-ethoxycarbonyl, oder 2-Triarylsilylethoxycarbonyl, wie 2-Triphenylsilylethoxycarbonyl.

Weitere, als Aminoschutzgruppen in Frage kommende Acylreste sind auch entsprechende Reste organischer Phosphor-, Phosphon- oder Phosphinsäuren, wie Diniederalkylphosphoryl, z.B. Dimethylphosphoryl, Diethylphosphoryl, Di-n-propylphosphoryl oder Diisopropylphosphoryl, Dicycloalkylphosphoryl, z.B.

Dicyclohexylphosphoryl, gegebenenfalls substituiertes Diphenylphosphoryl, z.B. Diphenylphosphoryl, gegebenenfalls, z.B. durch Nitro substituiertes Di-(phenylniederalkyl)-phosphoryl, z.B. Dibenzylphosphoryl oder Di-(4-nitrobenzyl)-phosphoryl, gegebenenfalls substituiertes Phenyloxyphenyl-phosphonyl, z.B. Phenyloxyphenyl-phosphonyl, Diniederalkylphosphinyl, z.B. Diethylphosphinyl, oder gegebenenfalls substituiertes Diphenylphosphinyl, z.B. Diphenylphosphinyl.

In einer Arylmethylaminogruppe, die eine Mono-, Di- oder insbesondere Triarylmethylaminogruppe darstellt, sind die Arylreste insbesondere gegebenenfalls substituierte Phenylreste. Solche Gruppen sind beispielsweise Benzyl-, Diphenylmethyl- und insbesondere Tritylamino.

Eine verätherte Mercaptogruppe in einer mit einem solchen Rest geschützten Aminogruppe ist in erster Linie Arylthio oder Arylniederalkylthio, worin Aryl insbesondere gegebenenfalls, z.B. durch Niederalkyl, wie Methyl oder tert.-Butyl, Niederalkoxy, wie Methoxy, Halogen, wie Chlor, und/oder Nitro substituiertes Phenyl ist. Eine entsprechende Aminoschutzgruppe ist z.B. 4-Nitrophenylthio.

In einem als Aminoschutzgruppe verwendbaren 2-Acyl-niederalk-1-en-1-ylrest ist Acyl z.B. der entsprechende Rest einer Niederalkancarbonsäure, einer gegebenenfalls, z.B. durch Niederalkyl, wie Methyl oder tert.-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters. Entsprechende Schutzgruppen sind in erster Linie 1-Niederalkanoyl-prop-1-en-2-yl, z.B. 1-Acetyl-prop-1 -en-2-yl, oder 1-Niederalkoxycarbonyl-prop-1-en-2-yl, z.B. 1-Ethoxycarbonyl-prop-1-en-2-yl.

Bevorzugte Aminoschutzgruppen sind Acylreste von Kohlensäurehalbestern, insbesondere tert.-Butyloxycarbonyl, gegebenenfalls, z.B. wie angegeben, substituiertes Benzyloxycarbonyl, z.B. 4-Nitrobenzyloxycarbonyl, oder Diphenylmethoxycarbonyl, oder 2-Halogen-niederalkoxycarbonyl, wie 2,2,2-Trichlorethoxycarbonyl, ferner Trityl oder Formyl.

Es können auch Schutzgruppen verwendet werden, die vicinale Hydroxy- und/oder Aminogruppen gemeinsam schützen, wobei solche Schutzgruppen nach dem Prinzip der "latent functionality" nach beendeter Reaktion auch in gewünschte Substituenten überführt werden können. Zum Beispiel können die Hydroxygruppe $R_3$ ($R_3$ = OH) in 1-Stellung und die Aminogruppe $R_4$ in 2-Stellung des Zuckerteils gemeinsam durch eine Schutzgruppe der Formel $R_8 - \overset{|}{C} =$ (VII) in Form eines 2-Oxazolins geschützt werden, worin $R_8$ Niederalkyl oder vorzugsweise Phenyl bedeutet. Das 2-Oxazolin erhält man nach verschiedenen an sich bekannten Verfahren. Man kann z.B. eine Verbindung der Formel VI mit geschützter Carboxylgruppe, worin $R_3$ Hydroxy und $R_4$ Benzoylamino bedeutet, zunächst mit HCl in Acetylchlorid ins 1-Chlorid überführen und die erhaltene Verbindung dann mit einem Silbersalz, z.B. $AgNO_3$, in Collidin behandeln.

Alternativ kann man eine Verbindung der Formel VI mit geschützter Carboxylgruppe, worin $R_4$ Benzoylamino bedeutet, in Aceton mit gasförmigen Chlorwasserstoff umsetzen.

Die Aufspaltung des so hergestellten 2-Oxazolinrings, die besonders zur Herstellung von N-Benzoyl-Verbindungen der Formel I ($R_4$ = Benzoylamino) geeignet ist, erfolgt mit verdünnter Säure, am besten bei einem pH-Wert von 2-4, z.B. 3, als Eintopfreaktion in an sich bekannter Weise, z.B. mit einem sauren Ionenaustauscher, insbesondere einem solchen mit Sulfonsäuregruppen, wie Amberlite IR-120 (ein Styrolharz mit stark sauren Sulfogruppen) oder Dowex 50 (Polystyrolsulfonsäuren) oder einer starken anorganischen oder organischen Säure, wie Salzsäure, Bromwasserstoffsäure, Schwefelsäure oder einer Sulfonsäure, z.B. Methansulfonsäure, oder einer gegebenenfalls im aromatischen Ring substituierten Phenylsulfonsäure, wie p-Toluolsulfonsäure, oder Trifluoressigsäure. Arbeitet man dabei in Gegenwart von Wasser, erhält man in 1-Stellung eine freie Hydroxygruppe. Arbeitet man dagegen in Gegenwart von Alkoholen, z.B. Methanol, erhält man Verbindungen, worin $R_3$ der Rest des betreffenden Alkohols ist.

Die vicinalen Hydroxygruppen $R_8$ und $R_7$ werden bevorzugterweise mittels einer gemeinsamen Alkyliden-, vorzugsweise Isopropyliden-, oder Benzylidengruppe geschützt.

Die Abspaltung der Schutzgruppen, die nicht Bestandteil des gewünschten Endprodukts der Formel I sind, erfolgt in an sich bekannter Weise, z.B. mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig.

Eine geschützte Aminogruppe setzt man in an sich bekannter und je nach Art der Schutzgruppen in verschiedenartiger Weise, vorzugsweise mittels Solvolyse oder Reduktion, frei. 2-Halogen-niederalkoxycarbonylamino (gegebenenfals nach Umwandlung einer 2-Brom-niederalkoxycarbonylaminogruppe in eine 2-Iod-niederalkoxycarbonylaminogruppe), Aroylmethoxycarbonylamino oder 4-Nitro-benzyloxycarbonylamino kann z.B. durch Behandeln mit einem geeigneten chemischen Reduktionsmittel, wie Zink in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagens, wie Natriumthiophenolat, und

4-Nitro-benzyloxycarbonylamino auch durch Behandeln mit einem Alkalimetall-, z.B. Natriumdithionit, gespalten werden. Gegebenenfalls substituiertes Diphenylmethoxycarbonylamino, tert.-Niederalkoxycarbonylamino oder 2-trisubstituiertes Silylethoxycarbonylamino kann durch Behandeln mit einer geeigneten Säure, z.B. Ameisen- oder Trifluoressigsäure, gegebenenfalls substituiertes Benzyloxycarbonylamino z.B. mittels Hydrogenolyse, d.h. durch Behandeln mit Wasserstoff in Gegenwart eines geeigneten Hydrierkatalysators, wie eines Palladiumkatalysators, gegebenenfalls substituiertes Triarylmethylamino oder Formylamino z.B. durch Behandeln mit einer Säure, wie Mineralsäure, z.B. Chlorwasserstoffsäure, oder einer organischen Säure, z.B. Ameisen-, Essig- oder Trifluoressigsäure, gegebenenfalls in Gegenwart von Wasser, gespalten werden, und eine mit einer organischen Silylgruppe geschützte Aminogruppe kann z.B. mittels Hydrolyse oder Alkoholyse freigesetzt werden. Eine durch 2-Halogenacetyl, z.B. 2-Chloracetyl, geschützte Aminogruppe kann durch Behandeln mit Thioharnstoff in Gegenwart einer Base, oder mit einem Thiolatsalz, wie einem Alkalimetallthiolat, des Thioharnstoffs und anschliessende Solvolyse, wie Alkoholyse oder Hydrolyse, des entstandenen Kondensationsprodukts freigesetzt werden. Eine durch 2-substituiertes Silylethoxycarbonyl geschützte Aminogruppe kann auch durch Behandeln mit einem Fluoridanionen liefernden Salz der Fluorwasserstoffsäure in die freie Aminogruppe übergeführt werden.

Eine durch eine geeignete Acylgruppe, eine organische Silylgruppe oder durch gegebenenfalls substituiertes 1-Phenylniederalkyl geschützte Hydroxygruppe wird analog einer entsprechend geschützten Aminogruppe freigesetzt. Durch gegebenenfalls substituiertes 1-Phenylniederalkyl, z.B. Benzyl, geschütztes Hydroxy wird vorzugsweise durch katalytische Hydrierung, z.B. in Gegenwart eine Palladium-auf-Kohle-Katalysators, freigesetzt. Eine durch 2,2-Dichloracetyl geschützte Hydroxygruppe wird z.B. durch basische Hydrolyse, eine durch tert.-Niederalkyl oder durch einen 2-oxa- oder 2-thia-aliphatischen oder -cycloaliphatischen Kohlenwasserstoffrest veretherte Hydroxygruppe durch Acidolyse, z.B. durch Behandeln mit einer Mineralsäure oder einer starken Carbonsäure, z.B. Trifluoressigsäure, freigesetzt. Mit einem organischen Silylrest, z.B. Trimethylsilyl, verethertes Hydroxy kann auch mit einem Fluoridanionen liefernden Salz der Fluorwasserstoffsäure, z.B. Tetrabutylaminoniumfluorid, freigesetzt werden.

Bevorzugt werden beim Vorhandensein von mehreren geschützten funktionellen Gruppen die Schutzgruppen so gewählt, dass gleichzeitig mehr als eine solche Gruppe abgespalten werden kann, beispielsweise acidolytisch, wie durch Behandeln mit Trifluoressigsäure oder Ameisensäure, oder reduktiv, wie durch Behandeln mit Zink und Essigsäure, oder mit Wasserstoff und einem Hydrierkatalysator, wie einem Palladium-Kohle-Katalysator. Wenn die gewünschten Endstoffe Schutzgruppen enthalten, z.B. wenn $R_3$ Benzyloxy bedeutet, werden diejenigen Schutzgruppen, die nach erfolgter Reaktion abgespalten werden sollen, so gewählt, dass sie regioselektiv wieder abgespalten werden können, z.B. kann eine durch Benzyloxycarbonyl geschützte Aminogruppe in 2-Stellung des Zuckerteils durch milde katalytische Hydrierung freigesetzt werden, wobei eine Benzyloxygruppe als Rest $R_3$ erhalten bleibt, weil deren Abspaltung härtere Reaktionsbedingungen, z.B. eine viel längere Reaktionszeit, erfordert.

Ein reaktionsfähiges Säurederivat einer Verbindung der Formel VI ist insbesondere ein reaktionsfähiger (aktivierter) Ester, ein reaktionsfähiges Anhydrid oder ein reaktionsfähiges cyclisches Amid.

Reaktionsfähige (aktivierte) Ester einer Säure der Formel VI sind insbesondere am Verknüpfungskohlenstoffatom des veresternden Restes ungesättigte Ester, z.B. vom Vinylester-Typ, wie eigentliche Vinylester (die man z.B. durch Umesterung eines entsprechenden Esters mit Vinylacetat erhalten kann; Methode des aktivierten Vinylesters), Carbamoylvinylester (die man z.B. durch Behandeln der entsprechenden Säure mit einem Isoxazoliumreagens erhalten kann; 1,2-Oxazolium- oder Woodward-Methode), oder 1-Niederalkoxyvinylester (die man z.B. durch Behandeln der entsprechenden Säure mit einem Niederalkoxyacetylen erhalten kann; Ethoxyacetylen-Methode), oder Ester vom Amidinotyp, wie N,N′-disubstituierte Amidinoester (die man z.B. durch Behandeln der entsprechenden Säure mit einem geeigneten N,N′-disubstituierten Carbodiimid, z.B. N,N′-Dicyclohexylcarbodiimid, erhalten kann; Carbodiimid-Methode), oder N,N-disubstituierte Amidinoester (die man z.B. durch Behandeln der entsprechenden Säure mit einem N,N-disubstituierten Cyanamid erhalten kann; Cyanamid-Methode), geeignete Arylester, insbesondere durch Elektronen-anziehende Substituenten geeignet substituierte Phenylester (die man z.B. durch Behandeln der entsprechenden Säure mit einem geeignet substituierten Phenol, z.B. 4-Nitrophenol, 4-Methylsulfonyl-phenol, 2,4,5-Trichlorphenol, 2,3,4,5,6-Pentachlor-phenol oder 4-Phenyldiazophenol, in Gegenwart eines Kondensationsmittels, wie N,N′-Dicyclohexylcarbodiimid, erhalten kann; Methode der aktivierten Arylester), Cyanmethylester (die man z.B. durch Behandeln der entsprechenden Säure mit Chloracetonitril in Gegenwart einer Base erhalten kann; Cyanmethylester-Methode), Thioester, insbesondere gegebenenfalls, z.B. durch Nitro, substituierte Phenylthioester (die man z.B. durch Behandeln der entsprechenden Säure mit gegebenenfalls, z. B. durch Nitro, substituierten Thiophenolen, u.a. mit Hilfe der Anhydrid- oder Carbodiimid-Methode, erhalten kann; Methode der aktivierten Thiolester), Amino- oder Amidoester (die man z.B. durch Behandeln der entsprechenden Säure mit einer N-Hydroxy-amino- bzw. N-Hydroxy-amido-Verbindung, z.B. N-Hydroxy-succinimid, N-Hydroxy-piperidin, N-Hydroxy-phthalimid oder 1-Hydroxy-benzotriazol, z.B. nach der Anhydrid- oder Carbodiimid-Methode, erhalten kann; Methode der aktivierten N-Hydroxyester) oder Silylester (die man z.B. durch Behandeln der entsprechenden Säure mit einem Silylierungsmittel, z.B. Hexamethyldisilazan, erhalten kann und die leicht mit Hydroxy-, nicht aber mit Aminogruppen reagieren).

Anhydride einer Säure der Formel VI können symmetrische oder vorzugsweise gemischte Anhydride dieser Säure sein, so z.B. Anhydride mit anorganischen Säuren, wie Säurehalogenide, insbesondere Säurechloride (die man z.B. durch Behandeln der entsprechenden Säure mit Thionylchlorid, Phosphorpentachlorid oder Oxalylchlorid erhalten kann; Säurechloridmethode), Azide (die man z.B. aus einem entsprechenden Säureester über das entsprechende Hydrazid und dessen Behandlung mit salpetriger Säure erhalten kann; Azidmethode), Anhydride mit Kohlensäurehalbderivaten, wie mit entsprechenden Estern, z.B. Kohlensäureniederalkylhalbestern (die man z.B. durch Behandeln der entsprechenden Säure mit Halogen-, wie Chlorameisensäure-niederalkylestern oder mit einem 1-Niederalkoxycarbonyl-2-niederalkoxy- 1,2-dihydro-chinolin, z.B. 1-Niederalkoxycarbonyl-2-ethoxy-1,2-dihydrochinolin, erhalten kann; Methode der gemischten O-Alkyl-kohlensäureanhydride), oder Anhydride mit dihalogenierter, insbesondere dichlorierter Phosphorsäure (die man z.B. durch Behandeln der entsprechenden Säure mit Phosphoroxychlorid erhalten kann; Phosphoroxychloridmethode), oder Anhydride mit organischen Säuren, wie gemischte Anhydride mit organischen Carbonsäuren (die man z.B. durch Behandeln der entsprechenden Säure mit einem gegebenenfalls substituierten Niederalkan- oder Phenylalkancarbonsäurehalogenid, z.B. Phenylessigsäure-, Pivalinsäure- oder Trifluoressigsäurechlorid erhalten kann; Methode der gemischten Carbonsäureanhydride) oder mit organischen Sulfonsäuren (die man z.B. durch Behandeln eines Salzes, wie eines Alkalimetallsalzes, der entsprechenden Säure, mit einem geeigneten organischen Sulfonsäurehalogenid, wie Niederalkan- oder Aryl-, z.B. Methan- oder p-Toluolsulfonsäurechlorid, erhalten kann; Methode der gemischten Sulfonsäureanhydride), sowie symmetrische Anhydride (die man z.B. durch Kondensation der entsprechenden Säure in Gegenwart eines Carbodiimids oder von 1-Diethylaminopropin erhalten kann; Methode der symmetrischen Anhydride).

Geeignete cyclische Amide sind insbesondere Amide mit fünfgliedrigen Diazacyclen aromatischen Charakters, wie Amide mit Imidazolen, z.B. Imidazol (die man z.B. durch Behandeln der entsprechenden Säure mit N,N′-Carbonyldiimidazol erhalten kann; Imidazolid-Methode), oder Pyrazolen, z.B. 3,5-Dimethyl-pyrazol (die man z.B. über das Säurehydrazid durch Behandeln mit Acetylaceton erhalten kann; Pyrazolid-Methode).

Derivate von Säuren der Formel VI, die als Acylierungsmittel verwendet werden, können auch in situ gebildet werden. So kann inan z.B. N,N′-di-substituierte Amidinoester in situ bilden, indem man das Gemisch des Ausgangsmaterials der Formel V und der als Acylierungsmittel verwendeten Säure in Gegenwart eines geeigneten N,N-disubstituierten Carbodiimids, z.B. N,N′-Dicyclohexylcarbodiimid, zur Reaktion bringt. Ferner kann man Amino- oder Amidoester der als Acylierungsmittel verwendeten Säuren in Gegenwart des zu acylierenden Ausgangsmaterials der Formel V bilden, indem man das Gemisch der entsprechenden Säure- und Amino-Ausgangsstoffe in Gegenwart eines N,N′-disubstituierten Carbodiimids, z.B. N,N′-Dicyclohexyl-carbodiimid, und eines N-Hydroxy-amins oder N-Hydroxy-amids, z.B. N-Hydroxysuccinimid, gegebenenfalls in Anwesenheit einer geeigneten Base, z.B. 4-Dimethylamino-pyridin, umsetzt.

Die Reaktion kann in an sich bekannter Weise durchgeführt werden, wobei die Reaktionsbedingungen in erster Linie davon abhängen, ob und wie die Carboxylgruppe des Acylierungsmittels der Formel VI aktiviert ist, üblicherweise in Gegenwart eines geeigneten Lösungs- oder Verdünnungsmittels oder eines Gemisches von solchen, und, falls notwendig, in Gegenwart eines Kondensationsmittels, das z.B., wenn die an der Reaktion teilnehmende Carboxylgruppe als Anhydrid vorliegt, auch ein Säure-bindendes Mittel sein kann, unter Kühlen oder Erwärmen, z.B. in einem Temperaturbereich von etwa -30°C bis etwa +150°C, insbesondere von etwa 0°C bis +100°C, vorzugsweise von Raumtemperatur (ca. +20°C) bis +70°C, in einem offenen oder geschlossenen Reaktionsgefäss und/oder in der Atmosphäre eines Inertgases, z.B. Stickstoff. Uebliche Kondensationsmittel sind z.B.

Carbodiimide, beispielsweise N,N′-Diethyl-, N,N′-Dipropyl-, N,N′-Dicyclohexyl- oder N-Ethyl-N′(3-dimethylaminopropyl)-carbodiimid, geeignete Carbonylverbindungen, beispielsweise Carbonyldiimidazol, oder 1,2-Oxazoliumverbindungen, z.B. 2-Ethyl-5-phenyl-1,2-oxazolium-3′-sulfonat und 2-tert.-Butyl-5-methyl-isoxazolium-perchlorat, oder eine geeignete Acylaminoverbindung, z.B. 2-Ethoxy-1-ethoxycarbonyl-1,2-dihydrochinolin. Uebliche säurebindende Kondensationsmittel sind z.B. Alkalimetallcarbonate oder -hydrogencarbonate, z.B. Natrium- oder Kaliumcarbonat oder -hydrogencarbonat (üblicherweise zusammen mit einem Sulfat), oder organische Basen, wie üblicherweise sterisch gehinderte Triniederalkylamine, z.B. N,N-Diisopropyl-N-ethyl-amin.

Eine erhaltene Verbindung der Formel I kann in eine andere Verbindung der Formel I überführt werden, z.B. durch Acylierung von freiem Hydroxy $R_6$, $R_7$, $R_4$ oder $R_3$ oder freiem Amino $R_4$, $R_6$ oder $R_7$ oder durch Abspaltung einer Schutzgruppe, wie Benzyl im Rest $R_3$ oder einem Niederalkoxycarbonyl- oder Benzyloxycarbonylrest im Rest $R_4$, $R_6$ oder $R_7$, oder durch Ueberführung von Azido $R_6$ oder $R_7$ in Amino.

Die Acylierung von freiem Hydroxy $R_6$, $R_7$, $R_4$ oder $R_3$ oder freiem Amino $R_4$, $R_6$ oder $R_7$ wird analog der oben beschriebenen Acylierung einer Verbindung der Formel V mit einer Verbindung der Formel VI oder einem reaktionsfähigen Säurederivat davon durchgeführt. Auch die Abspaltung der Schutzgruppen erfolgt, wie oben beschrieben.

Die Ueberführung von Azido $R_6$ oder $R_7$ in Amino wird reduktiv durchgeführt. Geeignet ist jedes Reduktionsmittel, mit dem die Ueberführung eines Azids in ein Amin gelingt und das normale, d.h. unaktivierte, Ester- oder Amidbindungen unter den Reaktionsbedingungen nicht angreift. Besonders bevorzugt ist Triphenylphosphin oder Wasserstoff in Gegenwart eines geeigneten Katalysators, z.B eines Palladium-, Raney-Nickel- oder Platinkatalysators. Man kann jedoch auch Reduktionsmittel, wie Zink in Essigsäure, Aluminiumamalgam in einem feuchten Ether, Titan(III)-chlorid, Zinn(II)-chlorid/NaOH oder komplexe Bor- oder Aluminiumhydride, z.B. Natriumboranat, Lithiumalanat oder Natrium-bis-[2-methoxy-ethoxy]-di-hydrido-aluminat, verwenden.

Wenn sowohl $R_6$ als auch $R_7$ für Azido steht, kann man, z.B. mit Triphenylphosphin, in Abhängigkeit von den Reaktionsbedingungen entweder beide Azidogruppen oder selektiv nur die Azidogruppe $R_7$ in 6-Stellung des Zuckerteils reduzieren. Zur Reduktion der 6-Azido-Gruppe mit Triphenylphosphin verwendet man stöchiometrische Mengen oder einen leichten, z.B. 25 %igen Ueberschuss Phosphin und lässt vorteilhafterweise etwa 1-30 Stunden in einem inerten Lösungsmittel, z.B. einem cyclischen Ether, wie insbesondere absolutem Tetrahydrofuran, bei etwa 20°-40°C reagieren. Die Reduktion der 4- und 6-Azidogruppen gelingt in absolutem Tetrahydrofuran oder in einem Tetrahydrofuran-Dimethylformamid-Gemisch mit einem z.B. 50- bis 125%igen Ueberschuss Triphenylphosphin während 25-50 Stunden bei 50-70°C. Im Anschluss an die Reduktion mit Triphenylphosphin fügt man zur Zersetzung des entstandenen Phosphinimins konzentrierte Ammoniaklösung zu und rührt mehrere, z.B. 7 bis 50 Stunden bei Raumtemperatur.

Die katalytische Reduktion wird vorzugsweise in Alkohol-Wasser-Gemischen, z.B. Methanol-Wasser-Gemischen, unter Zusatz von etwas Säure, z.B. Essigsäure oder Salzsäure, bei einer Temperatur zwischen etwa +5°C und +70°C, und erforderlichenfalls unter Druck durchgeführt.

Die Reduktion mit den komplexen Hydriden wird vorzugsweise bei -30°C bis +20°C, z.B. 0°C, in einem inerten Lösungsmittel, z.B. einem geeigneten Ether, durchgeführt.

Die bei der Azidreduktion erzielten isolierten Reinausbeuten betragen etwa 75 bis 95 % der Theorie. Die Azidreduktion wird bei Temperaturen zwischen -30°C und +70°C in einem geeigneten Lösungsmittel und erforderlichenfalls unter Druck oder Schutzgas, z.B Stickstoff oder Argon, durchgeführt.

Säureadditionssalze von Verbindungen der Formel I erhält man in üblicher Weise, z.B. durch Behandeln mit einer Säure oder einem geeigneten Anionenaustauschreagens.

Säureadditionssalze können in üblicher Weise in die freien Verbindungen überführt werden, z.B. durch Behandeln mit einem geeigneten basischen Mittel.

Gemische von Isomeren können in an sich bekannter Weise, z.B. durch fraktionierte Kristallisation, Chromatographie etc. in die einzelnen Isomeren aufgetrennt werden.

Die Ausgangsstoffe der Formeln V und VI sind bekannt oder können nach an sich bekannten Verfahren hergestellt werden. Der Ausgangsstoff der Formel V, worin $R_1$ für Wasserstoff steht, d.h. Staurosporin, ist kommerziell erhältlich und kann durch Fermentation mit dem Stamm Streptomyces staurosporeus hergestellt werden. Dieser Stamm wurde unter der Nummer FERM P-3725 beim Fermentation Research Institute, Japan, im Zusammenhang mit der geprüften japanischen Patentveröffentlichung [Examined Patent Publication, Kokoku] Nr. 57-53076 hinterlegt, die am 11. 11. 1982 veröffentlicht wurde. Staurosporinderivate der Formel V, worin $R_1$ von Wasserstoff verschieden ist, sind z.B. beschrieben von 1. Takahashi et al., J. Pharmacol. Exp. Ther. 255(3) (1990) 1218-1221. Muraminsäurederivate der Formel VI sind z.B. beschrieben im britischen Patent 1570625, welches dem französischen Patent 2361902, der deutschen Offenlegungsschrift 2655500 und der japanischen Patentanmeldung mit der Anmeldenummer 147903/76 äquivalent ist, sowie im deutschen Patent 2450355 und im US-Patent 4,256,735. Verbindungen der Formel VI, worin $R_3$ für Wasserstoff steht, sind z.B. beschrieben im US-Patent 4,315,913. Die Verbindungen der Formel VIII sind z.B. beschrieben in der Europäischen Patentanmeldung mit der Anmeldenummer 84400413.5, welche am 12. 9. 1984 unter der Publikationsnummer 0118364 veröffentlicht wurde. Generell erhält man die Verbindungen der Formel VI, indem man das entsprechende Zucker-, 1-Desoxyzucker-, 1-Desoxyaminozucker- oder Aminozuckerderivat mit freier 3-Hydroxy-Gruppe mit einer Verbindung der Formel Hal-CH($R_5$)-COOH, worin Hal für eine Abgangsgruppe, wie insbesondere für geeignetes Halogen, wie Chlor, steht, in Gegenwart einer starken Base, wie Natriumhydrid, umsetzt, z.B. wie im Beispielteil beschrieben.

Die oben beschriebenen Verfahren, inklusive die Verfahren zur Abspaltung von Schutzgruppen und die zusätzlichen Verfahrensmassnahmen, werden, wenn nicht anders angegeben, in an sich bekannter Weise, z.B. in An- oder Abwesenheit von vorzugsweise inerten Lösungs- und Verdünnungsmitteln, wenn notwendig, in Anwesenheit von Kondensationsmitteln oder Katalysatoren, bei erniedrigter oder erhöhter Temperatur, z.B. in einem Temperaturbereich von etwa -20°C bis etwa 150°C, insbesondere von etwa 0°C bis etwa +70°C, vorzugsweise von etwa +10°C bis etwa +50°C, hauptsächlich bei Raumtemperatur, in einem geeigneten Gefäss und erforderlichenfalls in einer Inertgas-, z.B. Stickstoffatmosphäre, durchgeführt.

Dabei sind unter Berücksichtigung aller im Molekül befindlichen Substituenten, wenn erforderlich, z.B. bei Anwesenheit leicht hydrolysierbarer Reste, besonders schonende Reaktionsbedingungen, wie kurze Reaktionszeiten, Verwendung von milden sauren oder basischen Mitteln in niedriger Konzentration, stöchiometrische Mengenverhältnisse, Wahl geeigneter Katalysatoren, Lösungsmittel, Temperatur- und/oder Druckbedingungen,anzuwenden.

Die Erfindung betrifft auch diejenigen Ausführungsformen des Verfahrens, bei denen man von einer auf irgendeiner Stufe des Verfahrens als Zwischenprodukt erhältlichen Verbindung ausgeht und die fehlenden Verfahrensschritte durchführt oder das Verfahren auf irgendeiner Stufe abbricht oder einen Ausgangsstoff unter den Reaktionsbedingungen bildet oder in Form eines reaktionsfähigen Derivats oder Salzes verwendet. Dabei geht man vorzugsweise von solchen Ausgangsstoffen aus, die verfahrensgemäss zu den oben als besonders wertvoll beschriebenen Verbindungen führen.

Neue Ausgangsstoffe und/oder Zwischenprodukte sowie Verfahren zu ihrer Herstellung sind ebenfalls Gegenstand der vorliegenden Erfindung. Vorzugsweise werden solche Ausgangsstoffe verwendet und die Reaktionsbedingungen so gewählt, dass man zu den in dieser Anmeldung als besonders bevorzugt aufgeführten Verbindungen gelangt.

Die Erfindung betrifft auch die Verwendung der Verbindungen der Formel I und ihrer pharmazeutisch verwendbaren Säureadditionssalze, vorzugsweise in Form pharmazeutischer Zusammensetzungen, zur therapeutischen Behandlung des menschlichen oder tierischen Körpers, insbesondere im Falle der obengenannten Krankheiten. Die Erfindung betrifft auch ein Verfahren zur Hemmung der Proteinkinase C in einem Warmblüter, der einer solchen Behandlung bedarf , wobei man diesem Warmblüter eine effektive Proteinkinase C hemmende Dosis einer Verbindung der Formel I oder eines pharmazeutisch verwendbaren Säureadditionssalzes davon verabreicht. Die Dosierung des Wirkstoffs hängt unter anderem von der Art der Krankheit, der Art und Grösse der zu behandelnden Spezies, der Abwehrlage des Organismus und der Applikationsweise ab. Beispielsweise verabreicht man an einen Warmblüter von etwa 70 kg Körpergewicht eine tägliche Dosis von 1 mg bis 1500 mg, hauptsächlich von 100 mg bis 1000 mg, vorzugsweise von 200 mg bis 800 mg, z.B. 500 mg, einer Verbindung der Formel I. Diese Gesamt-Tagesdosis wird vorzugsweise auf 2-3 tägliche Applikationen verteilt. Dabei liegt die Dosierung bei oraler Applikation etwa zwei- bis dreimal höher als bei parenteraler Applikation, also eher im oberen Bereich der angegebenen Dosierungen.

Die Erfindung betrifft auch pharmazeutische Präparate, die eine wirksame Menge, insbesondere eine zur Prophylaxe oder Therapie einer der obengenannten Krankheiten wirksame Menge, der Aktivsubstanz zusammen mit pharmazeurisch verwendbaren Trägerstoffen enthalten, die sich zur topischen, enteralen, z.B. oralen oder rektalen, oder parenteralen Verabreichung eignen, und anorganisch oder organisch, fest oder flüssig sein können. Zur oralen Verabreichung verwendet man insbesondere Tabletten oder Gelatinekapseln, welche den Wirkstoff zusammen mit Verdünnungsmitteln, z.B. Lactose, Dextrose, Sukrose, Mannitol, Sorbitol, Cellulose und/oder Glycerin, und/oder Schmiermitteln, z.B. Kieselerde, Talk, Stearinsäure oder Salze davon, wie Magnesium- oder Calciumstearat, und/oder Polyethylenglykol, enthalten. Tabletten können ebenfalls Bindemittel, z.B. Magnesiumaluminiumsilikat, Stärken, wie Mais-, Weizen- oder Reisstärke, Gelatine, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel enthalten. Ferner kann man die pharmakologisch wirksamen Verbindungen der vorliegenden Erfindung in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Drucks und/oder Puffer enthalten. Die vorliegenden pharmazeutischen Präparate, die, wenn erwünscht, weitere pharmakologisch wirksame Stoffe, wie Antibiotika, enthalten können, werden in an sich bekannter Weise, z.B. mittels konventioneller Misch-, Granulier-, Dragier-, Lösungs- oder Lyophilisierungsverfahren, hergestellt und enthalten von etwa 0,01 % bis 90 %, im Fall von Lyophilisaten bis 100 %, insbesondere von etwa 0,1 % bis etwa 50 %, in erster Linie zwischen 1 % und 30 %, des bzw. der Aktivstoffe, wobei eine Wirkstoffkonzentration unterhalb von 1% insbesondere f ür topisch zu applizierende Präparate geeignet ist.

Die nachfolgenden Beispiele illustrieren die Erfindung, ohne sie in irgendeiner Form einzuschränken. Die $R_f$-Werte werden auf Kieselgeldünnschichtplatten (Firma Merck, Darmstadt, Deutschland) ermittelt. Das Verhältnis der Laufmittel in den verwendeten Laufmittelgemischen zueinander ist in Volumenanteilen (V/V), Temperaturen sind in Grad Celsius angegeben. Die Konzentration, c, der Substanz im Lösungsmittel(gemisch) ist

im Falle der optischen Drehung in Prozent (Gewicht/Volumen) angegeben.

Abkürzungen:

DCCI Dicyclohexylcarbodiimid
HOBT 1-Hydroxybenztriazol

Beispiel 1:

3,74 g (3,99 mMol) N-(1-α-O-Benzyl-4,6-O-benzyliden-2-N-acetyl-muramyl)-staurosporin, das noch 0,95 Mol Wasser enthält, werden in 100 ml 80%iger Essigsäure suspendiert und 4,5 Stunden bei 60° gerührt. Die so erhaltene gelbe Lösung giesst man danach auf 500 ml Eiswasser, rührt die farblose Suspension 1 Stunde bei Raumtemperatur, nutscht die ausgefallenen Kristalle ab und wäscht diese mit Wasser. Das so erhaltene Rohprodukt reinigt man durch Säulenchromatographie an 300 g Kieselgel Si 60 (Merck 7734, 0,063 - 0,2 mm) in Chloroform-Ethanol (98 : 2; 15 ml Fraktionen). Die Fraktionen 476 - 590 werden vereinigt und im Hochvakuum bei 30° eingedampft. Nach Umkristallisation des Rückstandes aus 200 ml Essigsäureethylester erhält man N-(1-α-O-Benzyl-2-N-acetyl-muramyl)-staurosporin als beige Kristalle vom Smp. 224 - 226° (ab 220° Sintern), die noch 0.82 Mol Wasser enthalten; $[\alpha]_D^{20}$ = +188,1 ± 2,1° (c = 0,472; Methanol), $R_f$ = 0,12 (Chloroform : Ethanol = 95 : 5), $R_f$ = 0,48 (Chloroform : Methanol = 9 : 1).
$C_{46}H_{49}O_{10}$ . 0,82 $H_2O$ (846,70)
Ber. C 65,25 H 6,03 N 8,27 O 20,45 $H_2O$ 1,74
Gef. C 65,17 H 6,10 N 8,33 O 20,32 $H_2O$ 1,74
Das Ausgangsmaterial erhält man wie folgt:

Stufe 1.1:

Zu einer Lösung von 4,71 g (10 mMol) 1-α-O-Benzyl-4,6-O-benzyliden-2-N-acetyl-muraminsäure in 200 ml absolutem N,N'-Dimethylformamid gibt man bei 0° 1,65 g (11 mMol) 1-Hydroxybenzotriazol und 2,27 g (11 mMol) N,N'-Dicyclohexylcarbodiimid und rührt 3,5 Stunden bei 0°. Anschliessend werden 4,20 g (9 mMol) Staurosporin zugegeben und die so erhaltene Lösung 1,5 Stunden bei 0° und 21 Stunden bei Raumtemperatur gerührt. Danach gibt man zwecks vollständigem Umsatz nochmals eine Aktivesterlösung aus 1,57 g (3,3 mMol) 1-α-O-Benzyl-4,6-O-benzyliden-2-N-acetyl-muraminsäure, 0,55 g (3,7 mMol) 1-Hydroxybenzotriazol und 0,76 g (3,7 mMol) N,N'-Dicyclohexylcarbodiimid zu und rührt weitere 21 Stunden bei Raumtemperatur. Die so erhaltene gelbliche Suspension versetzt man mit 300 ml Wasser, rührt 1,5 Stunden bei Raumtemperatur, nutscht die ausgefallenen Kristalle ab und wäscht mit Wasser nach. Die Wasserphase wird verworfen.Das Nutschgut wird in 100 ml Methylenchlorid suspendiert, 1 Stunde bei Raumtemperatur gerührt, filtriert, mit Methylenchlorid nachgewaschen und das Filtrat im Hochvakuum bei 30° zur Trockne eingedampft. Den Rückstand (gelbes Harz) reinigt man durch Säulenchromatographie an 1 kg Kieselgel Si 60 (Merk 7754, 0,063 - 0,2 mm) in Chloroform-Ethanol (98 : 2; 25 ml Fraktionen). Die Fraktionen 198 - 218 (DC-rein) und die Fraktionen 181 - 187, bzw. 219 - 235 (Mischfrakrionen) werden vereinigt und im Hochvakuum bei 30° zur Trochne eingedampft. Die Mischfraktionen werden nochmals an 320 g Kieselgel Si 60 (Merck 7754, 0,063 - 0,2 mm) in Chloroform-Ethanol (98 : 2; 10 ml Fraktionen) gereinigt Die Fraktionen 88 - 123 werden vereinigt und ebenfalls eingedampft. Nach Umkristallisation der so erhaltenen DC-reinen Fraktionen aus Ethanol erhält man N-(1-α-O-Benzyl-4,6-O-benzyliden-2-N-acetyl-muramyl)-staurosporin als beige Kristalle vom Smp. 214 - 216° (ab 210° Sintern), die noch 0,95 Mol Wasser enthalten; $[\alpha]_D^{20}$ = +228,6 ± 1,8° (c = 0,560; Methanol), $R_f$ = 0,44 (Chloroform : Ethanol = 95 : 5), $R_f$ = 0,85 (Chloroform : Methanol = 9 : 1).
$C_{53}H_{53}N_5O_{10}$. 0,95 $H_2O$ (937,15)
Ber. C 67,93 H 5,90 N 7,47 O 18,69 $H_2O$ 1,83
Gef. C 67,71 H 6,02 N 7,49 O 18,79 $H_2O$ 1,83

Beispiel 2:

275 mg (0,32 mMol) N-(1-α-O-Benzyl-2-N-acetyl-muramyl)-staurosporin (siehe Beispiel 1), das noch 0,82 Mol Wasser enthält, werden in 10 ml absolutem Methanol mit insgesamt 300 mg 5%iger Palladiumkohle (Degussa E101N) unter Normaldruck bei Raumtemperatur 69 Stunden katalytisch hydriert. Danach wird vom Katalysator abfiltriert und das Filtrat im Hochvakuum bei 30° zur Trockne eingedampft. Den Rückstand (gelber Schaum) löst man in 3 ml absolutem Methanol und versetzt diese Lösung mit 100 ml Essigsäureethylester.

Nach Zugabe von 100 ml n-Pentan wird 16 Stunden bei Raumtemperatur stehengelassen. Die dann ausgefallenen Kristalle werden abgesaugt und mit n-Pentan nachgewaschen. Nochmalige Kristallisation aus Methanol-Essigsäureethylester (1:10) ergibt N-(2-N-Acetyl-muramyl)-staurosporin (1-α,β-Anomerengemisch) als farblose Kristalle vom Smp. 257-259° (ab 254° Sintern), die noch 1,5 Mol Wasser enthalten; $[\alpha]_D^{20}$ = +195,5 ± 2,0° (c = 0,488; Methanol), $R_f$ = 0,44 (Chloroform : Methanol = 4 : 1), $R_f$ = 0,77 (Chloroform : Methanol : Wasser = 70 : 30 : 5).

$C_{39}H_{43}N_5O_{10}$ . 1,50 $H_2O$ (768,82)
Ber. C 60,93 H 6,03 N 9, 11 O 23,93 $H_2O$ 3,51
Gef. C 61,13 H 6,13 N 8,94 O 23,81 $H_2O$ 3,52

Beispiel 3:

130 ml (theor. 5 mMol) einer Suspension von rohem N-(4,6-O-Isopropyliden-2-N-acetyl-muramyl)-staurosporin (1-α,β-Anomerengemisch) in N,N'-Dimethylformamid werden mit 260 ml 60%iger Essigsäure versetzt und 18 Stunden bei Raumtemperatur gerührt. Dann wird abgenutscht (N,N'-Dicyclohexylharnstoff) und die Mutterlauge am Hochvakuum bei 40° eingedampft. Den Rückstand suspendiert man in 100 ml Wasser und rührt die so erhaltene Suspension 1,5 Stunden bei Raumtemperatur. Die ausgefallenen Kristalle werden abgesaugt und mit Wasser nachgewaschen. Das Filtrat wird verworfen. Das Rohprodukt reinigt man durch Flashchromatographie bei 0,4 bar an 500 g Kieselgel Si 60 (Merck 9385, 0,063 - 0,040 mm) in Chloroform-Methanol (9 : 1; 25 ml Fraktionen). Die Fraktionen 60 - 120 werden vereinigt und im Hochvakuum bei 30° zur Trockne eingedampft. Den Rückstand löst man in 30 ml Methanol und filtriert die so erhaltene Lösung durch ein Milliporefilter (Fluoropore, FGLP, 0,2 μm). Das Filtrat wird im Hochvakuum bei 30° zur Trockne eingedampft, der Rückstand in 10 ml heissem Methanol aufgenommen und die so erhaltene Lösung mit 200 ml heissem Essigsäureethylester versetzt. Die so entstehende Suspension wird unter Rühren auf Raumtemperatur abgekühlt und 1 Stunde bei 0° nachgerührt Die ausgefallenen Kristalle werden abgenutscht und mit Essigsäureethylester nachgewaschen. Man erhält N-(2-N-Acetyl-muramyl)-staurosporin als 1-α,β-Anomerengemisch in Form beiger Kristalle vom Smp. 256 - 258°, die noch 1,29 Mol Wasser enthalten; $[\alpha]_D^{20}$ = +198,7 ± 1,9° (c = 0,536; Methanol), $R_f$ = 0,16 (Chloroform : Methanol = 9 : 1), $R_f$ = 0,44 (Chloroform : Methanol = 4 : 1), $R_f$ = 0,75 (Chloroform : Methanol : Wasser = 70 : 30 : 5).

$C_{39}H_{43}N_5O_{10}$ . 1,29 $H_2O$ (765,04)
Ber. C 61,23 H 6,01 N 9,15 O 23,61 $H_2O$ 3,04
Gef. C 61,20 H 5,94 N 9,37 O 23,43 $H_2O$ 3,03

Das Ausgangsmaterial erhält man folgendermassen:

Stufe 3.1:

Zu einer Lösung von 4,11 g (10 mMol; 2,43 mMol/g) 4,6-O-Isopropyliden-2-N-acetyl-muraminsäure-natriumsalz (1-α,β-Anomerengemisch) in 30 ml Wasser tropft man bei 0° 10 ml 1 N Salzsäure, verdünnt dann mit 50 ml N,N'-Dimethylformamid und dampft im Hochvakuum bei 30° zur Trockne ein. Nach zweimaligem Abdampfen mit je 50 ml N,N'Dimethylformamid erhält man 4,6-O-Isopropyliden-2-N-acetyl-muraminsäure (1-α,β-Anomerengemisch), die ohne weitere Reinigung in 200 ml absolutem N,N'-Dimethylformamid suspendiert wird. Zu dieser Suspension gibt man bei 0° 1,80 g (12 mMol) 1-Hydroxybenzotriazol und 3,09 g (15 mMol) N,N'-Dicyclohexylcarbodiimid und rührt diese farblose Suspension 2 Stunden bei 0°. Danach werden 2,33 g (5 mMol) Staurosporin zugegeben und noch 1 Stunde bei 0° und 20 Stunden bei Raumtemperatur weitergerührt. Anschliessend gibt man zwecks vollständigem Umsatz des Staurosporins nochmals wie oben beschrieben eine Aktivesterlösung aus 2,11 g (5 mMol) 4,6-O-Isopropyliden-2-N-acetyl-muraminsäure-natriumsalz (Darstellung der freien Säure wie beschrieben), 0,9 g (6 mMol) 1-Hydroxybenzotriazol und 1,545 g (7,5 mMol) N,N'-Dicyclohexylcarbodiimid zu (Reaktionsdauer 2 Stunden) und rührt weitere 8 Stunden bei Raumtemperatur bis zum vollständigen Umsatz des Staurosporins. Nach einer Gesamtreaktionszeit von 30 Stunden wird die erhaltene gelbliche Suspension auf 130 ml eingeengt und das in dieser enthaltene rohe 4,6-O-Isopropyliden-2-N-acetyl-muramyl-staurosporin (1-α,β-Anomerengemisch) ohne weitere Reinigung zur Darstellung von 2-N-Acetyl-muramyl-staurosporin verwendet.

Beispiel 4:

Zu einer Lösung von 1,97 g (2,33 mMol) N-(1-α-O-Benzyl-2-N-acetyl-muramyl)-staurosporin, das noch 0,82 Mol Wasser enthält, in 19 ml absolutem Pyridin tropft man bei -15° innerhalb von 10 Minuten eine Lösung

von 0,339 g [2,95 mMol; 230 μl (d = 1,474)] Methansulfochlorid in 19 ml absolutem Pyridin. Die so erhaltene braune Lösung wird 1 Stunde bei - 15°, 2 Stunden bei 0° und 20 Stunden bei Raumtemperatur gerührt. Dann wird nochmals bei - 15° innerhalb von 3 Minuten eine Lösung von 31 μl (0,4 mMol) Methansulfochlorid in 3 ml absolutem Pyridin zugetropft und weitere 10,5 Stunden bei -15° bis Raumtemperatur gerührt. Danach giesst man die Lösung auf 300 ml Eiswasser, dekantiert das Lösungsmittel von ausgefallenem Rohprodukt ab und verrührt den Rückstand mit 200 ml Diethylether. Die so erhaltenen Kristalle werden abgenutscht und mit Diethylether nachgewaschen. Das Rohprodukt reinigt man durch Säulenchromatographie an 200 g Kieselgel Si 60 (Merck 7734; 0,063 - 0,2 mm) in Chloroform-Ethanol (98 : 2; 15 ml Fraktionen). Die Fraktionen 185 - 340 werden vereinigt und im Hochvakuum bei 30° zur Trockne eingedampft. Nach Umkristallisation aus Essigsäureethylester-n-Pentan (2:1; insgesamt 150 ml) erhält man N-(6-O-Mesyl- 1-α-O-benzyl-2-N-acetyl-muramyl)-staurosporin als schwach gelbgefärbte Kristalle vom Smp. 218 - 219° (ab 215° Sintern), die noch 0,93 Mol Wasser enthalten; $[\alpha]_D^{20}$ + 184,1 ± 1,9° (c = 0,527; Methanol), $R_f$ = 0,28 (Chloroform: Ethanol = 95 : 5), $R_f$ = 0,88 (Chloroform: Methanol = 9 : 1).

$C_{47}H_{51}N_5O_{12}S$. 0,93 $H_2O$ (926,77)
Ber. C 60,91 H 5,75 N 7,56 O 22,32 S 3,46 $H_2O$ 1,81
Gef. C 61,00 H 5,91 N 7,53 O 22,10 S 3,44 $H_2O$ 1,81

Beispiel 5:

Zu einer Lösung von 971 mg (1,05 mMol) N-(6-O-Mesyl-1-α-O-benzyl-2-N-acetyl-muramyl)-staurosporin, das noch 0,93 Mol Wasser enthält, in 13 ml absolutem N,N'-Dimethylformamid gibt man bei Raumtemperatur unter Rühren 638 mg (9,8 mMol) Natriumazid (reinst). Die so erhaltene gelbliche Suspension wird 6 Stunden bei 75° gerührt, dann bei Raumtemperatur mit 250 ml Chloroform verdünnt und dreimal mit je 100 ml Wasser gewaschen. Die Chloroformphasen werden vereinigt, über Natriumsulfat getrocknet, filtriert und im Hochvakuum bei 30° zur Trockne eingedampft. Den Rückstand (gelbes Harz) löst man in 50 ml heissem Essigsäureethylester und versetzt diese Lösung mit 50 ml heissem n-Pentan. Die nach 16 Stunden bei Raumtemperatur ausgefallenen Kristalle werden abgenutscht und mit n-Pentan nachgewaschen. Man erhält N-(6-Azido-1-α-O-benzyl-2-N-acetyl-6-desoxy-muramyl)-staurosporin als schwach gelb gefärbte Kristalle vom Smp. 213 - 216° (ab 202° Sintern), die noch 0,76 Mol Wasser enthalten; $[\alpha]_D^{20}$ = +189,5 ± 2,1° (c = 0,478; Chloroform : Methanol = 1 : 1), $[\alpha]_D^{20}$ = +189,3 ± 2,0° (c = 0,495; Methanol), $R_f$ = 0,17 (Chloroform : Ethanol = 95 : 5), $R_f$ = 0,44 (Chloroform : Methanol = 9 : 1), $R_f$ = 0,77 (Chloroform : Methanol = 4 : 1).

$C_{46}H_{48}N_8O_9$. 0,76 $H_2O$ (870,63)
Ber. C 63,46 H 5,73 N 12,87 O 17,94 $H_2O$ 1,57
Gef. C 63,16 H 5,69 N 12,82 O 17,95 $H_2O$ 1,57

Beispiel 6:

Zu einer Lösung von 770 mg (0,88 mMol) N-(6-Azido-1-α-O-benzyl-2-N-acetyl-6-desoxy-muramyl)-staurosporin, das noch 0,76 Mol Wasser enthält, in 25 ml absolutem Tetrahydrofuran (puriss.) gibt man 354 mg (1,35 mMol) Triphenylphosphin (puriss.) und rührt 12 Stunden bei 45°. Danach wird die so erhaltene Lösung mit 1,35 ml 25%iger Ammoniaklösung versetzt und 21 Stunden bei Raumtemperatur weitergerührt. Danach wird mit 250 ml Chloroform verdünnt und dreimal mit je 100 ml Wasser gewaschen. Die Chloroformphasen werden vereinigt, über Natriumsulfat getrocknet, filtriert und im Hochvakuum bei 30° zur Trockne eingedampft. Der Rückstand (gelblicher Schaum) wird durch Flashchromatographie bei 0,3 bar an 250 g Kieselgel Si 60 (Merck 9385; 0,063 - 0,040 mm) in Chloroform-Methanol (4 : 1; 15 ml Fraktionen) gereinigt. Die Fraktionen 58 - 100 werden vereinigt und im Hochvakuum bei 30° eingedampft. Den Rückstand löst man in 20 ml Methanol, versetzt diese Lösung mit 630 μl einnormaler Methansulfonsäure in Ethanol und gibt schliesslich 100 ml Essigsäureethylester hinzu. Die so entstehende Suspension rührt man 0,5 Stunden bei Raumtemperatur und 1,5 Stunden bei 0°, saugt die ausgefallenen Kristalle ab und wäscht sie mit Essigsäureethylester. Nach nochmaliger Umkristallisation aus Methanol-Essigsäureethylester (3 : 20) erhält man N-(6-Amino-1-α-O-benzyl-2-N-acetyl-6-desoxy-muramyl)-staurosporin-methansulfonat als schwach gelbe Kristalle vom Smp. 239 -242° (Zers., ab 235° Sintern), die noch 2,86 Mol Wasser enthalten; $[\alpha]_D^{20}$ = +172,1 ± 2,0° (c = 0,488; Methanol), $R_f$ = 0,15 (Chloroform : Methanol = 4 : 1), $R_f$ = 0,59 (Chloroform : Methanol : Wasser = 70 : 30 : 5).

$C_{46}H_{50}N_6O_9$. $CH_3SO_3H$. 2,86 $H_2O$ (978,57)
Ber. C 57,69 H 6,15 N 8,59 O 24,30 S 3,28 $H_2O$ 5,27
Gef. C 57,84 H 5,86 N 8,67 O 24,19 S 3,13 $H_2O$ 5,27

Beispiel 7:

0,435 g (0,44 mMol) N-(6-Amino-1-α-0-benzyl-6-desoxy-2-N-acetyl-muramyl)-staurosporin-methansulfonat, das noch 2,86 Mol Wasser enthält, werden in 60 ml absolutem Methanol mit insgesamt 480 mg 5%iger Palladiumkohle (Degussa E101N) unter Normaldruck bei Raumtemperatur 87 Stunden katalytisch hydriert. Danach wird vom Katalysator abfiltriert und das Filtrat im Hochvakuum bei 30° zur Trockne eingedampft. Den gelben, kristallinen Rückstand reinigt man durch Säulenchromatographie an 30 g Kieselgel Si 60 (Merck; 0,063 - 0,200 mm) in Chloroform-Methanol-Wasser (70 : 30 : 5; 5 ml Fraktionen). Die Fraktionen 18 - 45 werden vereinigt und im Hochvakuum bei 30° zur Trockne eingedampft. Nach Umkristallisation des Rückstands aus Methanol-Essigsäureethylester (3 : 20) erhält man N-(6-Amino-6-desoxy-2-N-acetyl-muramyl)-staurosporin (1-α,β-Anomerengemisch) im Gemisch mit seinem Methansulfonat (enthält 0,25 Mol Methansulfonsäure) als beige Kristalle, die sich ab 225° zersetzen und noch 2,17 Mol Wasser enthalten; $[\alpha]_D^{20}$ = +177,0 ± 2,1° (c = 0,469; Methanol), $R_f$ = 0,25 (Chloroform : Methanol : Wasser = 70 : 30 : 5), $R_f$ = 0,29 (n-Butanol : Essigsäure : Wasser = 67 : 10 : 23), $R_f$ = 0,53 (Essigsäureethylester : n-Butanol : Pyridin : Essigsäure : Wasser = 42 : 21 : 21 : 6 : 10).

Beispiel 8:

355 mg (0,38 mMol) N-(6-O-Mesyl-1-α-O-benzyl-2-N-acetyl-muramyl)-staurosporin, das noch 0,93 Mol Wasser enthält, werden in 30 ml Methanol-Chloroform (28:2) mit insgesamt 500 mg 5%iger Palladiumkohle (Degussa E101N) unter Normaldruck bei Raumtemperatur 117 Stunden katalytisch hydriert. Danach wird vom Katalysator abfiltriert und das Filtrat im Hochvakuum bei 30° zur Trockne eingedampft. Den Rückstand reinigt man durch Säulenchromatographie an 30 g Kieselgel Si 60 (Merck 7734; 0,063 - 0,2 mm) in Chloroform-Ethanol (95 : 5; 10 ml Fraktionen). Die Fraktionen 35 - 85 werden vereinigt und im Hochvakuum bei 30° zur Trockne eingedampft. Nach Umkristallisation des Rückstands aus Essigsäureethylester erhält man N-(6-O-Mesyl-2-N-acetyl-muramyl)-staurosporin (1-α,β-Anomerengemisch) als beige Kristalle, die sich ab 170° zersetzen und noch 1,28 Mol Wasser enthalten; $[\alpha]_D^{20}$ = +171,6 ± 1,9° (c = 0,514; Methanol), $[\alpha]_D^{20}$ = + 185,0 ± 2,0° (c = 0,50; Dimethylformamid), $R_f$ = 0,11 (Chloroform : Ethanol = 95 : 5), $R_f$ = 0,41 (Chloroform : Methanol = 9: 1 ), $R_f$ = 0,81 Chloroform : Methanol = 4 : 1).
$C_{40}H_{45}N_5O_{12}S$ . 1,28 $H_2O$ (842,95)
Ber. C 57,00 H 5,69 N 8,31 O 25,21 S 3,80 $H_2O$ 2,74
Gef. C 56,99 H 5,60 N 8,05 O* S 3,61 $H_2O$ 2,74 (*nicht bestimmt)

Beispiel 9:

40 ml einer Suspension von rohem N-(4,6-O-Isopropyliden-2-N-acetyldesmethylmuramyl-staurosporin (siehe Stufe 9.2; theor. 1 mMol) werden mit 80 ml 60%iger Essigsäure versetzt und 23 Stunden bei Raumtemperatur gerührt. Nach Filtration der so erhaltenen gelben Suspension wird das Filtrat im Hochvakuum bei 30° eingedampft und der Rückstand mit 50 ml Wasser 2 Stunden bei Raumtemperatur gerührt. Das ausgefallene Rohprodukt wird abgesaugt und durch Flashchromatographie bei 0,25 bar an 100 g Kieselgel Si 60 (Merck 9385; 0,063 - 0,040 mm) in Chloroform-Methanol (9 : 1; 20 ml Fraktionen) gereinigt. Die Fraktionen 40 - 65 werden vereinigt und im Hochvakuum bei 30° zur Trockne eingedampft. Den Rückstand löst man in 30 ml Methanol, versetzt die so erhaltene Lösung mit Akrivkohle, erhitzt zum Sieden und filtriert die noch warme Suspension durch ein Milliporefilter (Fluoropore, FGLP, 0,2 μm). Das fast farblose Filtrat wird im Vakuum bei 30° auf ca. 3 ml eingeengt, mit 10 ml Essigsäureethylester versetzt und erhitzt. Nach dem Abkühlen erhält man N-(2-N-Acetyl-desmethylmuramyl)-staurosporin (1-α,β-Anomerengemisch) als beige Kristalle vom Smp. 224 - 226° (ab 208° Sintern), die noch 1,60 Mol Wasser enthalten; $[\alpha]_D^{20}$ = +153,5 ± 2,0° (c = 0,495; Dimethylformamid), $R_f$ = 0,10 (Chloroform : Methanol = 9 : 1 ), $R_f$ = 0,43 (Chloroform : Methanol = 4 : 1), $R_f$ = 0,73 (Chloroform : Methanol : Wasser = 70 : 30 : 5).
$C_{38}H_{41}N_5O_{10}$. 1,60 $H_2O$ (756,60)
Ber. C 60,33 H 5,89 N 9,26 O 24,53 $H_2O$ 3,81
Gef. C 60,13 H 5,91 N 9,28 O 24,30 $H_2O$ 3,81.

Das Ausgangsmaterial erhält man folgendermassen:

Stufe 9.1:

5,0 g (10,75 mMol) 1-α-O-Benzyl-4,6-O-isopropyliden-2-N-acetyl-desmethylmuraminsäure-natriumsalz

werden in 50 ml Wasser bei pH 7,0 mit 1,0 g 5%iger Palladiumkohle (Degussa E101N) unter Normaldruck 20 Stunden bei Raumtemperatur katalytisch hydriert. Danach wird vom Katalysator abfiltriert und das Filtrat im Hochvakuum bei 40° zur Trockne eingedampft. Man erhält rohes 4,6-O-Isopropyliden-2-N-acetyl-desmethylmuraminsäure-natriumsalz (1-α,β-Anomerengemisch) als farblosen Schaum, der ohne weitere Reinigung verarbeitet wird; $R_f$ = 0,23 (Chloroform : Methanol : Wasser = 70 : 30 : 5), $R_f$ = 0,71 (Acetonitril : Wasser = 3 : 1).

Stufe 9.2:

Eine Lösung von 714 mg (ca. 2 mMol) rohem 4,6-O-Isopropyliden-2-N-acetyl-desmethylmuraminsäure-natriumsalz (1-α,β-Anomerengemisch) in 10 ml Wasser wird bei 0° mit 2 ml einnormaler Salzsäure versetzt. Die so erhaltene Lösung wird mit 40 ml N,N'-Dimethylformamid verdünnt und im Hochvakuum bei 40° zur Trockne eingedampft. Der Rückstand wird noch zweimal in je 40 ml N,N'-Dimethylformamid suspendiert und erneut eingedampft. Die so erhaltene rohe 4,6-O-Isopropyliden-2-N-acetyl-desmethylmuraminsäure (1-α,β-Anomerengemisch) wird in 40 ml absolutem N,N'-Dimethylformamid suspendiert. Zu dieser Suspension gibt man bei 0° 450 mg (3 mMol) 1-Hydroxybenzotriazol und 825 mg (4 mMol) N,N'-Dicyclohexylcarbodiimid und rührt 2 Stunden bei 0°. Danach gibt man 466 mg (1 mMol) Staurosporin zu und rührt anschliessend 1 Stunde bei 0° und 20 Stunden bei Raumtemperatur. Die so erhaltene gelbliche Suspension wird danach direkt zu N-(2-N-Acetyl-desmethylmuramyl)-staurosporin umgesetzt (Abspaltung der 4,6-O-Isopropylidengruppe).

Beispiel 10:

Eine Suspension von 1,588 g (1,71 mMol) N-(1-α-O-Benzyl-4,6-O-benzyliden-2-N-acetyl-L-muramyl)-staurosporin, das noch 0,45 Mol Wasser enthält, in 35 ml Methanol wird nach Zusatz von 0,23 g (0,908 mMol) Iod (doppelt sublimiert) 1 Stunde unter Rühren und Rückfluss erhitzt. Die dann erhaltene orangerote Suspension wird im Hochvakuum bei 40° zur Trockne eingedampft und der Rückstand in 200 ml Chloroform aufgenommen. Die Chloroformlösung wird zweimal mit je 50 ml 0,1-normaler Natriumthiosulfatlösung und einmal mit 50 ml Wasser gewaschen. Die Chloroformphasen werden vereinigt, über Natriumsulfat getrocknet, filtriert und erneut eingedampft. Den Rückstand (1,66 g) reinigt man durch Säulenchromatographie an 250 g Kieselgel Si 60 (Merck, 0,063 - 0,2 mm) in Chloroform-Ethanol (98 : 2; 15 ml Fraktionen). Die Fraktionen 93 - 150 werden vereinigt und eingedampft. Nach Umkristallisation des Rückstands aus Essigsäureethylester erhält man N-(1-α-O-Benzyl-2-N-acetyl-L-muramyl)-staurosporin als beige Kristalle vom Smp. 230 - 232° (ab 224° Sintern), die noch 1,13 Mol Wasser enthalten; $[\alpha]_D^{20}$ = +117,4 ± 2,1° (c = 0,477; Methanol), $R_f$ = 0,28 (Chloroform : Ethanol = 95 : 5), $R_f$ = 0,55 (Chloroform : Methanol = 9 : 1).
$C_{46}H_{49}N_5O_{10}$ . 1,13 $H_2O$ (852,28)
Ber. C 64,83 H 6,06 N 8,22 O 20,89 $H_2O$ 2,39
Gef. C 65,12 H 6,04 N 8,34 O 20,69 $H_2O$ 2,39.

Beispiel 11:

Analog Beispiel 2 wird N-(2-N-Acetyl-L-muramyl)-staurosporin (1-α,β-Anomerengemisch) durch Hydrierung (23 Std. bei 46° C) von 200 mg N-(1-α-O-Benzyl-2-N-acetyl-L-muramyl)-staurosporin (Pd-C 5 %, Degussa E101N) hergestellt. Das Rohprodukt wird durch Kieselgelchromatographie gereinigt. Es werden 55 mg als amorphes, farbloses Pulver vom Smp. 250 - 255° Zers. erhalten; $R_f$ = 0,17 (Essigsäure-ethylester : Methanol = 9 : 1).

Das Ausgangsmaterial erhält man wie folgt:

Stufe 11.1:

Analog Beispiel 1, Stufe 1.1 erhält man aus 2,357 g (5 mMol) 1-α-O-Benzyl-4,6-O-benzyliden-2-N-acetyl-L-muraminsäure, 0,826 g (5,5 mMol) 1-Hydroxybenzotriazol, 1,135 g (5,5 mMol) N,N'-Dicyclohexylcarbodiimid und 1,866 g (4 mMol) Staurosporin in 100 ml N,N'-Dimethylformamid nach einer Reaktionszeit von insgesamt 22 Stunden (5 Stunden bei 0°, 17 Stunden bei Raumtemperatur), säulenchromatographischer Aufarbeitung [350 g Kieselgel Si 60, Merck 7754, 0,063 - 0,2 mm, in Chloroform-Ethanol (98 : 2)] und Kristallisation aus Essigsäureethylester N-(1-α-O-Benzyl-4,6-O-benzyliden-2-N-acetyl-L-muramyl)-staurosporin als farblose Kristalle vom Smp. 220 - 222° (ab 216° Sintern), die noch 0,45 Mol Wasser enthalten; $[\alpha]_D^{20}$ = +103,7 ± 2,2° (c = 0,463; Methanol), $R_f$ = 0,30 (Chloroform : Ethanol = 95 : 5), $R_f$ = 0,65 (Chloroform : Methanol = 9 : 1).
$C_{53}H_{53}N_5O_{10}$ . 0,45 $H_2O$ (928,14)

Ber. C 68,59 H 5,85 N 7,55 O 18,01 $H_2O$ 0,87,
Gef. C 68,90 H 5,92 N 7,67 O 17,75 $H_2O$ 0,88.

Beispiel 12:

Analog Beispiel 4 erhält man aus 1,145 g (1,34 mMol) N-(1-α-O-Benzyl-2-N-acetyl-L-muramyl)-staurosporin, das noch 1,13 Mol Wasser enthält, 0,198 g [1,72 mMol, 134,7 µl (d = 1,454)] Methansulfochlorid (puriss.) in 11,5 ml absolutem Pyridin nach einer Reakrionszeit von 19 Stunden ohne weiteren Zusatz von Methansulfochlorid und säulenchromatographischer Reinigung an 100 g Kieselgel Si 60 (Merck 7734, 0,063 - 0,2 mm) in Chloroform-Ethanol (98 : 2; 10 ml Fraktionen) nach Umkristallisation aus Essigsäureethylester N-(6-O-Mesyl-1-α-O-benzyl-2-N-acetyl-L-muramyl)-staurosporin als gelbliche Kristalle vom Smp. 220 - 221° (ab 215° Sintern), die noch 1,19 Mol Wasser enthalten; $[\alpha]_D^{20}$ +83,7 ± 2,0° (c = 0,490; Methanol-Chloroform 1 : 1), $R_f$ = 0,35 (Chloroform : Ethanol = 95 : 5), $R_f$ = 0,75 (Chloroform : Methanol = 9 : 1).
$C_{47}H_{51}N_5O_{12}S$ . 1,19 $H_2O$ (931,45)
Ber. C 60,61 H 5,78 N 7,52 O 22,66 S 3,44 $H_2O$ 2,30,
Gef. C 60,61 H 5,72 N 7,54 O 22,46 S 3,39 $H_2O$ 2,30.

Beispiel 13:

Analog Beispiel 5 erhält man aus 1,16 g (1,25 mMol) N-(6-O-Mesyl-1-α-O-benzyl-2-N-acetyl-L-muramyl)-staurosporin, das noch 1,19 Mol Wasser enthält, und 766 mg (11,78 mMol) Natriumazid (reinst) in 16 ml absolutem N,N'-Dimethylformamid nach einer Reaktionszeit von 8 Stunden bei 75° und Umkristallisation aus Essigsäureethylester-n-Pentan (1 : 1) N-(6-Azido-1-α-O-benzyl-2-N-acetyl-6-desoxy-L-muramyl)-staurosporin als beige Kristalle vom Smp. 214-216° (ab 208° Sintern), die noch 0,69 Mol Wasser enthalten; $[\alpha]_D^{20}$ = + 76,6 ± 1,9° (c = 0,522; Chloroform-Methanol 1 : 1), $R_f$ = 0,41 (Chloroform : Ethanol = 95 : 5), $R_f$ = 0,62 (Chloroform : Methanol = 9 : 1), $R_f$ = 0,73 (Chloroform : Methanol = 4 : 1).
$C_{46}H_{48}N_8O_9$. 0,69 $H_2O$ (869,37)
Ber. C 63,55 H 5,73 N 12,89 O 17,83 $H_2O$ 1,43
Gef. C 63,42 H 5,75 N 12,86 O 18,03 $H_2O$ 1,43.

Beispiel 14:

Analog Beispiel 6 erhält man aus 841 mg (0,97 mMol) N-(6-Azido-1-α-O-benzyl-2-N-acetyl-6-desoxy-L-muramyl)-staurosporin, das noch 0,69 Mol Wasser enthält, und 390 mg (1,48 mMol) Triphenylphosphin (puriss.) in 28 ml absolutem Tetrahydrofuran nach einer Reaktionszeit von 12 Stunden bei 45°, Behandlung mit 1,47 ml 25 %iger Ammoniaklösung (6 Stunden, Raumtemperatur), Flashchromatographie bei 0,3 bar an 250 g Kieselgel Si 60 (Merck 9385, 0,63 - 0,04 mm) in Chloroform-Methanol (4 : 1; 25 ml Fraktionen), sowie nachfolgender Ueberführung des freien Amins in das Methansulfonat und Umkristallisation aus Methanol-Essigsäureethylester (3 : 20) N-(6-Amino- 1-α-O-benzyl-2-N-acetyl-6-desoxy-L-muramyl)-staurosporin-methansulfonat als schwach gelbgefärbte Kristalle von Smp. 239 - 241° (Zers., ab 237° Sintern), die noch 2,3 Mol Wasser enthalten; $[\alpha]_D^{20}$ = +110,4 ± 2,2° (c = 0,462; Methanol), $R_f$ = 0,19 (Chloroform : Methanol = 4 :1 ), $R_f$ = 0,70 (Chloroform : Methanol : Wasser = 70 : 30 : 5).

Beispiel 15:

Analog Beispiel 7 erhält man aus 405 mg (0,42 mMol) N-(6-Amino-1-α-O-benzyl-2-N-acetyl-6-desoxy-L-muramyl)-staurosporin-methansulfonat, das noch 2,3 Mol Wasser enthält, durch katalytische Hydrierung mit insgesamt 580 mg 5%iger Palladiumkohle (Degussa E101N) nach einer Hydrierdauer von 92 Stunden, säulenchromatographischer Reinigung [25 g Kieselgel Si 60, Merck 7734, 0,063 - 0,2 mm, in Chloroform-Methanol-Wasser (70 : 30 : 5)] und Kristallisation aus Methanol-Essigsäureethylester (1 : 10) N-(6-Amino-2-N-acetyl-6-desoxy-L-muramyl)-staurosporin (1-α,β-Anomerengemisch), das als Gemisch von freiem Amin und Methansulfonat vorliegt (enthält 0,3 Mol Methansulfonsäure), als beige Kristalle, die sich ab 218° zersetzen und noch 3,4 Mol Wasser enthalten; $[\alpha]_D^{20}$ = +75,7 ± 4,2° (c = 0,235; Methanol), $R_f$ = 0,26 (n-Butanol : Essigsäure : Wasser = 67 : 10 : 23), $R_f$ = 0,48 (Essigsäureethylester : n-Butanol : Pyridin : Essigsäure : Wasser = 42 : 21 : 21 : 6 : 10).

Beispiel 16:

Analog Beispiel 8 erhält man aus 350 mg (0,376 mMol) N-(6-O-Mesyl-1-α-O-benzyl-2-N-acetyl-L-muramyl)-staurosporin, das noch 1,19 Mol Wasser enthält, durch katalyrische Hydrierung [450 mg 5%ige Palladiumkohle (Degussa E101N)] in Methanol (Hydrierdauer 68 Stunden) und Umkristallisation aus Methanol-Chloroform (1 : 2) N-(6-O-Mesyl-2-N-acetyl-L-muramyl)-staurosporin als α-Anomeres in Form farbloser Kristalle, die sich ab 192° zersetzen und noch 1,3 Mol Wasser enthalten; $[\alpha]_D^{20}$ = +111,1 ± 2,1° (c = 0,468; Dimethylformamid), $R_f$ = 0,37 (Chloroform : Methanol = 9 : 1), $R_f$ = 0,60 (Chloroform : Methanol = 4 : 1), $R_f$ = 0,88 (Chloroform : Methanol : Wasser = 70 : 30 : 5).

$C_{40}H_{45}N_5O_{12}S$ . 1,30 $H_2O$ (843,31)
Ber. C 56,97 H 5,69 N 8,30 O 25,23 S 3,80 $H_2O$ 2,78,
Gef. C 56,89 H 5,72 N 8,09 O 25,22 S 3,68 $H_2O$ 2,77.

Beispiel 17:

80 mg N-(1-α-O-Methyl-4,6-O-benzyliden-2-N-acetyl-desmethylmuramyl)-staurosporin in 2 ml Essigsäure und 1,2 ml $H_2O$ werden zur Entfernung der Benzyliden-Schutzgruppe während 2 Stunden bei 55° gerührt. Das Reaktionsgemisch wird dann unter Rühren auf 50 ml Essigsäureethylester-Hexan (1 : 1) gegossen. Der Niederschlag von ausgefallenem rohem N-(1-α-O-Methyl-2-N-acetyl-desmethylmuramyl)-staurosporin wird abfiltriert, wieder in Essigsäureethylester gelöst und nochmals mit Essigester-Hexan (1 : 1) gefällt. Um Benzaldehyd zu entfernen, wird das Produkt mehrere Male in Diethylether digeriert. Es wird N-(1-α-O-Methyl-2-N-acetyl-desmethylmuramyl)-staurosporin als amorphes, farbloses Pulver vom Smp. 247° erhalten. $R_f$ = 0.27 (Essigsäureethylester : Methanol = 9 : 1), $[\alpha]_D^{20}$ = + 169,1 ± 2,1° (c = 0,460; Methanol).

Das Ausgangsmaterial erhält man folgendermassen:

Stufe 17.1:

Eine Mischung von 729 mg (1,89 mMol) 1-α-O-Methyl-4,6-O-benzyliden-2-N-acetyl-desmethylmuraminsäure, 20 ml $CHCl_3$, 10 ml Isopropanol, 0,5 ml $H_2O$, 506 mg (2,45 mMol) DCCI und 376 mg (2,45 mMol) HOBT wird während 1 Stunde bei Raumtemperatur gerührt und anschliessend mit 1,05 g (2,26 mMol) Staurosporin versetzt. Die Reaktionsmischung wird während 20 Stunden bei Raumtemperatur gerührt und dann zur Trockne eingedampft. Der Rückstand wird in 200 ml Essigsäureethylester gelöst, zweimal mit gesättigter NaCl-Lösung gewaschen, getrocknet und zur Trockne eingedampft. Kieselgelchromatographie des Rohprodukts ergibt die gewünschte Verbindung, die noch DCCI als Verunreinigung enthält, und durch wiederholtes Digerieren in wenig kaltem $CH_2Cl_2$ und Abfiltrieren des unlöslichen DCCI gereinigt wird. Zum Schluss wird das Produkt in wenig $CH_2Cl_2$ gelöst und mit Hexan gefällt. Es wird N-(1-α-O-Methyl-4,6-O-benzyliden-2-N-acetyl-desmethylmuramyl)-staurosporin als farbloses, amorphes Pulver vom Smp. 235° erhalten; $R_f$ = 0,25 (Essigester : Methanol = 9 : 1).

Beispiel 18:

N-(1-α-O-Benzyl-4,6-O-isopropyliden-2-N-acetyl-desmethylmuramyl)-staurosporin wird analog Beispiel 2, Stufe 2.1 aus 300 mg (0,73 mMol) 1-α-O-Benzyl-4,6-O-isopropyliden-2-N-acetyl-desmethylmuraminsäure und 409 mg (0,88 mMol) Staurosporin in Form eines amorphen, farblosen Pulvers hergestellt; $R_f$ = 0,35 (Essigsäureethylester : Methanol = 98 : 2 und 0,27 (Toluol : Essigsäureethylester = 6 : 4). Das erhaltene Zwischenprodukt kann z.B. analog Beispiel 9 weiterverarbeitet werden.

Beispiel 19:

Analog Beispiel 2, Stufe 2.1 wird aus 940 mg (2,22 mMol) 1-α-O-Benzyl-4,6-O-isopropyliden-2-N-acetyl-L-muraminsäure, 460 mg (2,22 mMol) DCCI, 333 mg (2,22 mMol) HOBT und 940 mg (2,22 mMol) Staurosporin in 50 ml DMF 420 mg N-(1-α-O-Benzyl-4,6-O-isopropyliden-2-N-acetyl-L-muramyl)-staurosporin als amorphes, farbloses Pulver hergestellt; $R_f$ = 0,53 ($CH_2Cl_2$ : Methanol = 9 : 1), $R_f$ = 0,42 (Essigsäurethylester : Methanol = 95 : 5), $[\alpha]_D^{20}$ = + 151,0 ± 1,7 (c = 0,6; Methanol). Das erhaltene Zwischenprodukt kann z.B. analog Beispiel 9 weiterverarbeitet werden.

Beispiel 20:

Analog Beispiel 1 erhält man aus 327 mg (0,343 mMol) N-(1-α-O-Benzyl-4,6-O-benzyliden-2-N-acetyl-homomuramyl)-staurosporin, das noch 0,99 Mol Wasser enthält, in 10 ml 80%iger Essigsäure nach 8 Stunden bei 60° und säulenchromatographischer Reinigung des Rohprodukts an 50 g Kieselgel Si60 (Merck 9385, 0,040 - 0,063 mm) in Chloroform-Ethanol (95:5; 15 ml Fraktionen) und Kristallisation der Fraktionen 35-65 aus Essigsäureethylester N-(1-α-O-Benzyl-2-N-acetyl-homomuramyl)-staurosporin als beige Kristalle vom Smp. 217-219° (ab 205° Sintern), die noch 1,22 Mol Wasser enthalten; $[\alpha]_D^{20}$ = + 182,2 ± 1,9° (c = 0,540; Chloroform : Methanol = 1 : 1).

Das Ausgangsmaterial erhält man wie folgt:

Stufe 20.1:

Analog Beispiel 1, Stufe 1.1 erhält man aus 486 mg (1,0 mMol) 1-α-O-Benzyl-4,6-O-benzyliden-2-N-acetyl-homomuraminsäure (beschrieben in Beispiel 4 der Deutschen Offenlegungsschrift Nr. 2655500), 180 mg (1,2 mMol) 1-Hydroxybenzotriazol, 247 mg (1,2 mMol) N,N'-Dicyclohexylcarbodiimid und 373 mg (0,8 mMol) Staurosporin in 20 ml N,N'-Dimethylformamid nach einer Reaktionszeit von insgesamt 52 Stunden [9 Stdn. bei 0°, 43 Stdn. bei Raumtemperatur; nach 5 Stdn. bei 0° und 24 Stdn. bei Raumtemperatur werden weitere 146 mg (0,3 mMol) 1-α-O-Benzyl-4,6-O-benzyliden-2-N-acetyl-homomuraminsäure, 54 mg (0,36 mMol) 1-Hydroxybenzotriazol und 75 mg (0,36 mMol) N,N'-Dicyclohexylcarbodiimid zwecks vollständigem Umsatz des eingesetzten Staurosporins zugegeben], säulenchromatographischer Reinigung (100 g Kieselgel Si60, Merck 9385, 0,040-0,063 mm) in Chloroform (15 ml Fraktionen) und Kristallisation der Fraktionen 103 - 180 aus Essigsäureethylester-n-Pentan (1 : 2) N-(1-α-O-Benzyl-4,6-O-benzyliden-2-N-acetyl-homomuramyl)-staurosporin als beige Kristalle vom Smp. 205 - 207° (ab 203°Sintern), die noch 0,99 Mol Wasser enthalten; $[\alpha]_D^{20}$ = + 231,5 ± 1,9° (c = 0,520; Methanol).

Beispiel 21:

Analog Beispiel 20 erhält man aus 347 mg (0,37 mMol) N-(1-α-O-Benzyl-4,6-O-benzyliden-2-N-acetyl-L-homomuramyl)-staurosporin, das noch 0,73 Mol Wasser enthält, in 10 ml 80%iger Essigsäure nach 4 Stunden bei 60°, säulenchromatographischer Reinigung des Rohprodukts an 70 g Kieselgel Si 60 (Merck 9385, 0,040 - 0,063 mm) in Chloroform-Methanol (99 : 1, 15 ml Fraktionen) und Kristallisation der Fraktionen 200 - 220 aus Essigsäureethylester N-(1-α-O-Benzyl-2-N-acetyl-L-homomuramyl)-staurosporin als beige Kristalle vom Smp. 229 - 231° (ab 225° Sintern), die noch 1,15 Mol Wasser enthalten; $[\alpha]_D^{20}$ = + 64,0 ± 2,0° (c = 0,50; Chloroform : Methanol = 1 : 1).

Das Ausgangsmaterial erhält man folgendermassen:

Stufe 21.1:

Analog Beispiel 20, Stufe 20.1 erhält man aus 486 mg (1,0 mMol) 1-α-O-Benzyl-4,6-O-benzyliden-2-N-acetyl-L-homomuraminsäure, 180 mg (1,2 mMol) 1-Hydroxy-benzotriazol, 247 mg (1,2 mMol) N,N'-Dicyclohexylcarbodiimid und 373 mg (0,8 mMol) Staurosporin in 20 ml N,N'-Dimethylformamid nach einer Reaktionszeit von insgesamt 53 Stunden (10 Stdn. bei 0°, 43 Stdn. bei Raumtemperatur; erneute Zugabe von aktivierter 1-α-O-Benzyl-4,6-O-benzyliden-2-N-acetyl-L-homomuraminsäure wie in Stufe 20.1 beschrieben), säulenchromatographischer Reinigung (120 g Kieselgel Si60, Merck 9385, 0,040 - 0,063 mm) in Chloroform (15 ml Fraktionen) und Kristallisation der Fraktionen 320 - 500 aus Essigsäureethylester-n-Pentan (1 : 2) N-(1-α-O-Benzyl-4,6-O-benzyliden-2-N-acetyl-L-homomuramyl)-staurosp orin als beige Kristalle vom Smp. 212 - 214° (ab 208° Sintern), die noch 0,73 Mol Wasser enthalten; $[\alpha]_D^{20}$ = + 96,1 ± 1,8° (c = 0,541; Methanol).

Beispiel 22:

Analog Beispiel 2 erhält man aus 245 mg (0,28 mMol) N-(1-α-O-Benzyl-2-N-acetyl-L-homomuramyl)-staurosporin, das noch 1,15 Mol Wasser enthält, durch katalytische Hydrierung in 20 ml Methanol mit insgesamt 400 mg 5%iger Palladiumkohle (Degussa E101N) unter Normaldruck bei Raumtemperatur nach einer Hydrierdauer von 93 Stunden und Kristallisarion des Rohprodukuts aus Methanol N-(2-N-Acetyl-L-homomuramyl)-staurosporin (1-α -Anomeres) als farblose Kristalle von Smp. 230 - 232° (ab 224° Sintern), die noch 1,01 Mol Wasser enthalten; $[\alpha]_D^{20}$ = + 116,1 ± 2,1° (c = 0,472; N,N'-Dimethylformamid).

Beispiel 23:

Zu einer Lösung von 200 mg (0,428 mMol) 1-α-O-Benzyl-4,6-O-diacetyl-2-N-acetyl-muraminsäure in 10 ml absolutem N,N'-Dimethylformamid gibt man bei 0° 77 mg (0,513 mMol) 1-Hydroxybenzotriazol und 106 mg (0,513 mMol) N,N'-Dicyclohexylcarbodiimid und rührt 3 Stunden bei 0°. Anschliessend werden 179 mg (0,385 mMol) Staurosporin zugegeben und die so erhaltene gelbliche Suspension 1,5 Stunden bei 0° und 26 Stunden bei Raumtemperatur gerührt. Danach gibt man zwecks vollständigem Umsatz des eingesetzten Staurosporins nochmals eine Aktivesterlösung aus 40 mg (0,086 mMol) 1-α-O-Benzyl-4,6-O-diacetyl-2-N-acetyl-muraminsäure, 17 mg (0,11 mMol) 1-Hydroxybenzotriazol und 22 mg (0,11 mMol) N,N'-Dicyclohexylcarbodiimid zu und rührt weitere 18 Stunden bei Raumtemperatur. Die so erhaltene gelbliche Suspension versetzt man mit 30 ml Wasser, rührt eine Stunde bei Raumtemperatur, nutscht die ausgefallenen Kristalle ab und wäscht mit Wasser nach. Die Wasserphase wird verworfen. Das Nutschgut wird in 10 ml Methylenchlorid suspendiert, 1 Stunde bei Raumtemperatur gerührt, filtriert, mit Methylenchlorid nachgewaschen und das Filtrat im Hochvakuum bei 30° zur Trockne eingedampft. Den Rückstand (gelbes Harz) reinigt man durch Säulenchromatographie an 40 g Kieselgel Si60 (Merck 9385, 0,040 - 0,063 mm) in Chloroform (10 ml Fraktionen). Die Fraktionen 175 - 305 werden vereinigt und im Hochvakuum bei 30° zur Trockne eingedampft. Nach Umkristallisallisation des Rückstands (beige Kristalle) aus Essigsäureethylester-Cyclohexan (1 : 2) erhält man N-(1-α-O-Benzyl-4,6-O-diacetyl-2-N-acetylmuramyl)-staurosporin als beige Kristalle vom Smp. 189,2 - 191,8° (ab 185° Sintern), die noch 0,48 Mol Wasser enthalten; $[\alpha]_D^{20}$ = + 175,7 ± 1,9° (c = 0,535; Chloroform).

Das Ausgangsmaterial erhält man wie folgt:

Stufe 23.1:

Zu einer Lösung von 4,09 g (8,64 mMol) 1-α-O-Benzyl-2-N-acetyl-muraminsäure-benzylester in 90 ml absolutem Pyridin gibt man 2,645 g (25,91 mMol; 2,45 ml) Essigsäureanhydrid (puriss., Fluka) und rührt 92 Stunden bei Raumtemperatur. Die so erhaltene gelbliche Lösung wird im Hochvakuum bei 40° auf ca. 20 ml eingeengt und dann mit 100 ml Wasser versetzt. Nach zweistündigem Rühren bei Raumtemperatur werden die ausgefallenen Kristalle abgenutscht, mit Wasser nachgewaschen und im Hochvakuum bei 50° getrocknet. Nach Umkristallisation aus 200 ml Cyclohexan erhält man 1-α-O-Benzyl-4,6-O-diacetyl-muraminsäure-benzylester als farblose Kristalle vom Smp. 102,2 - 103,4° (ab 100° Sintern); $[\alpha]_D^{20}$ = + 106,9 ± 1,0° (c = 1,024; Chloroform).

Stufe 23.2:

900 mg (1,6 mMol) 1-α-O-Benzyl-4,6-O-diacetyl-2-N-acetyl-muraminsäure-benzylester werden in 20 ml absolutem Methanol mit 200 mg 10%iger Palladiumkohle unter Normaldruck bei Raumtemperatur 40 Minuten katalytisch hydriert. Danach wird vom Katalysator abfiltriert und das Filtrat im Hochvakuum bei 30° zur Trockne eingedampft. Nach Umkristallisation des farblosen, kristallinen Rückstands aus 30 ml Cyclohexan-Essigsäureethylester (1 : 1) erhält man 1-α-O-Benzyl-4,6-O-diacetyl-2-N-acetyl-muraminsäure als farblose Kristalle vom Smp. 136,3 - 137,2° (ab 132° Sintern); $[\alpha]_D^{20}$ = + 122,5 ± 1,9° (c = 0,519; Chloroform).

Beispiel 24:

Analog Beispiel 23 erhält man aus 692 mg (ca. 1,0 mMol) roher 1-α-O-Benzyl-4-O-acetyl-6-O-stearoyl-2-N-acetyl-muraminsäure, 195 mg (1,3 mMol) 1-Hydroxybenzotriazol, 268 mg (1,3 mMol) N,N'-Dicyclohexylcarbodiimid und 370 mg (0,8 mMol) Staurosporin in 20 ml absolutem N,N'-Dimethylformamid nach einer Reaktionszeit von insgesamt 42 Stunden [4 Stdn. bei 0°, 38 Stdn. bei Raumtemperatur; nach 2 Stdn. bei 0° und 20 Stdn. bei Raumtemperatur werden weitere 173 mg (0,25 mMol) rohe 1-α-O-Benzyl-4-O-acetyl-6-O-stearoyl-2-N-acetyl-muraminsäure, 49 mg (0,325 mMol) 1-Hydroxybenzotriazol und 67 mg (0,325 mMol) N,N'-Dicyclohexylcarbodiimid zwecks vollständigem Umsatz des eingesetzten Staurosporins zugegeben], säulenchromatographischer Reinigung (140 g Kieselgel Si 60, Merck 9385, 0,040 - 0,063 mm) in Chloroform (15 ml Fraktionen) und Kristallisation der Fraktionen 140 - 240 aus 16,5 ml Cyclohexan-Essigsäureethylester (15 : 1,5) N-(1-α-O-Benzyl-4-O-acetyl-6-O-stearoyl-2-N-acetyl-muramyl)-staurosporin als beige Kristalle von Smp. 131,4 - 134,1° (ab 128° Sintern), die noch 0,45 Mol Wasser enthalten; $[\alpha]_D^{20}$ = + 152,5 ± 2,0° (c = 0,499; Chloroform).

Das Ausgangsmaterial erhält man folgendermassen:

Stufe 24.1:

Zu einer Lösung von 5,445 g (11,5 mMol) 1-α-O-Benzyl-2-N-acetyl-muraminsäure-benzylester in 110 ml absolutem Pyridin gibt man 4,529 g (14,95 mMol; 4,99 ml) Stearoylchlorid (pract., Fluka, 90 - 95%) und rührt die so erhaltene Lösung 24 Stunden bei Raumtemperatur. Die so erhaltene bräunliche Lösung wird im Hochvakuum bei 40° auf ca. 20 ml eingeengt, die danach erhaltene braune Suspension mit 100 ml Wasser versetzt, 2 Stunden bei Raumtemperatur gerührt und die Wasserphase abdekantiert.
Die Wasserphase wird verworfen. Der schmierige Rückstand wird in 100 ml Chloroform gelöst, über Natriumsulfat getrocknet, filtriert und im Hochvakuum bei 30° zur Trockne eingedampft.
Das Rohprodukt (beiges Harz) reinigt man durch Flashchromatographie bei 0,4 bar an 500 g Kieselgel Si 60 (Merck 9385, 0,040 - 0,063 mm) in Chloroform (20 ml Fraktionen). Die Fraktionen 135 - 250 werden vereinigt und im Hochvakuum bei 30° eingedampft. Man erhält 1-α-O-Benzyl-6-O-stearoyl-2-N-acetyl-muraminsäure-benzylester als leicht gelbliches Oel; $[\alpha]_D^{20}$ = + 65,5 ± 0,9° (c = 1,077; Chloroform).

Stufe 24.2:

Analog Beispiel 23, Stufe 23.1 erhält man aus 5,980 g (8,08 mMol) 1-α-O-Benzyl-6-O-stearoyl-2-N-acetyl-muraminsäure-benzylester und 1,65 g (16,2 mMol; 1,53 ml) Essigsäureanhydrid (puriss., Fluka) in 80 ml absolutem Pyridin nach einer Reaktionszeit von 44 Stunden bei Raumtemperatur und Umkristallisation des Rohprodukts aus 50 ml Methanol 1-α-O-Benzyl-4-O-acetyl-6-O-stearoyl-2-N-acetyl-muraminsäure-benzylester als farblose Kristalle vom Smp. 72,4 - 73,2° (ab 70° Sintern); $[\alpha]_D^{20}$ = + 80,9 ± 1,8° (c = 0,544; Chloroform).

Stufe 24.3:

Analog Beispiel 23, Stufe 23.2 erhält man aus 1,82 g (2,3 mMol) 1-α-O-Benzyl-4-O-acetyl-6-O-stearyl-2-N-acetyl-muraminsäure-benzylester durch katalytische Hydrierung unter Normaldruck bei Raumtemperatur in 40 ml Methanol mit 0,4 g 10%iger Palladiumkohle (Hydrierdauer 45 Minuten) und säulenchromatographischer Reinigung des Rohprodukts an 140 g Kieselgel Si 60 (Merck 9385, 0,040 - 0,063 mm) in Chloroform-Methanol-Wasser (70 : 30 : 5; 10 ml Fraktionen) nach Vereinigung der Fraktionen 21 - 36 1-α-O-Benzyl-4-O-acetyl-6-O-stearoyl-2-N-acetyl-muraminsäure als gelblichen Schaum, $R_f$ = 0,50 (Chloroform : Methanol = 7 : 3), der noch eine geringe Menge eines Nebenproduktes enthält ($R_f$-Wert in dem obengenannten Laufmittel = 0,40) und ohne weitere Reinigung verarbeitet wird.

Beispiel 25:

Eine Lösung von 302 mg (0,35 mMol) N-(1-Desoxy-4,6-O-benzyliden-2-N-acetyl-muramyl)-staurosporin, das noch 0,3 Mol Wasser und 0,5 Mol Essigsäureethylester enthält, in 12 ml 80%iger Essigsäure wird 7,5 Stunden bei 60° gerührt. Danach wird im Hochvakuum bei 40° zur Trockne eingedampft und der so erhaltene Rückstand (gelbe Kristalle) durch Säulenchromatographie an 30 g Kieselgel Si 60 (Merck 9385, 0,040 - 0,063 mm) in Chloroform-Ethanol (95 : 5; 10 ml Fraktionen) gereinigt. Die Fraktionen 85 - 175 werden vereinigt und im Hochvakuum bei 30° eingedampft. Nach Umkristallisation des Rückstands aus 30 ml Essigsäureethylester erhält man N-(1-Desoxy-2-N-acetyl-muramyl)-staurosporin als beige, schwach hygroskopische Kristalle vom Smp. 241 - 243° (ab 234° Sintern), die noch 1,24 Mol Wasser enthalten; $[\alpha]_D^{20}$ = + 198,4 ± 2,0° (c = 0,489; Methanol).

Das Ausgangsmaterial erhält man wie folgt:

Stufe 25.1:

Zu einer Suspension von 1,026 g (5,00 mMol) 1-Desoxy-2-N-acetyl-D-glucosamin [A. Hasegawa et al., Agric. Biol. Chem. **1986**, 50 (7), 1873] in 10 ml Benzaldehyd (puriss., Fluka) gibt man 1,02 g (7,5 mMol) Zinkchlorid (Merck, p.a.) und rührt 52 Stunden bei Raumtemperatur.

Die so erhaltene gelbe Lösung giesst man auf eine Mischung von 50 ml Eiswasser und 50 ml Toluol und rührt die so erhaltene gelbe Suspension 0,5 Stunden bei Raumtemperatur. Die ausgefallenen Kristalle werden abfiltriert und aus 30 ml Ethanol umkristallisiert. Man erhält 1-Desoxy-2-N-acetyl-4,6-O-benzyliden-D-glucosamin als farblose, schwach hygroskopische Kristalle vom Smp. 259 - 261° (ab 254° Sintern), die noch 0,16 Mol Wasser enthalten; $[\alpha]_D^{20}$ = - 53,4 ± 1,0° (c = 0,965; Chloroform : Methanol = 1 : 1).

Stufe 25.2:

Eine Suspension von 0,470 g (1,59 mMol) 1-Desoxy-2-N-acetyl-4,6-O-benzyliden-D-glucosamin, das noch 0,16 Mol Wasser enthält, in 50 ml absolutem 1,4-Dioxan (Merck, p.a.) versetzt man bei 65° mit 0,256 g (6,40 mMol) 60%igem Natriumhydrid in Oel (pract. Fluka). Die graue Suspension rührt man 2 Stunden unter Rückfluss, lässt wieder auf 65° abkühlen und tropft eine Lösung von 0,138 ml (1,60 mMol) S(-)-2-Chlorpropionsäure (puriss., Fluka) in 5 ml absolutem 1,4-Dioxan innerhalb von 4 Minuten zu.

Dieses Gemisch wird 5 Stunden unter Rückfluss und 17 Stunden bei Raumtemperatur gerührt, danach werden 20 ml Wasser zugetropft und im Hochvakuum bei 30° eingedampft. Den Rückstand nimmt man in 100 ml Wasser auf, säuert mit einnormaler Salzsäure an (pH1) und extrahiert zweimal mit je 150 ml Diethylether. Die organischen Phasen werden dreimal mit je 50 ml Wasser gewaschen, vereinigt, über Natriumsulfat getrocknet, filtriert und im Hochvakuum bei 30° eingedampft. Der Rückstand kristallisiert aus 10 ml Essigsäureethylester. Man erhält 1-Desoxy-4,6-O-benzyliden-2-N-acetyl-muraminsäure als farblose Kristalle vom Smp. 255 - 256° (ab 252° Sintern), die noch 0,04 Mol Wasser enthalten; $[\alpha]_D^{20}$ = - 8,6 ± 1,8° (c = 0,544; Methanol).

Stufe 25.3:

Eine bei 0° gehaltene Lösung von 0,388 g (1,06 mMol) 1-Desoxy-4,6-O-benzyliden-2-N-acetyl-muraminsäure, die noch 0,04 Mol Wasser enthält, in 10 ml N,N′-Dimethylformamid versetzt man mit 0,207 g (1,38 mMol) 1-Hydroxybenzotriazol und 0,285 g (1,38 mMol) N,N′-Dicyclohexylcarbodiimid und rührt 3 Stunden bei 0°. Zu der so erhaltenen farblosen Suspension gibt man 0,495 g (1,06 mMol) Staurosporin und rührt 1 Stunde bei 0° und 15 Stunden bei Raumtemperatur. Danach werden 40 ml Wasser hinzugefügt und weitere 0,5 Stunden bei Raumtemperatur gerührt. Die ausgefallenen Kristalle (N,N′-Dicyclohexylharnstoff) werden abgenutscht, mit Wasser nachgewaschen, im Vakuum getrocknet, in 20 ml Methylenchlorid suspendiert und 0,5 Stunden bei Raumtemperatur gerührt. Anschliessend werden die Kristalle (N,N′-Dicyclohexylharnstoff) erneut abfiltriert und das Filtrat im Hochvakuum bei 30° zur Trockne eingedampft. Den Rückstand reinigt man durch Säulenchromatographie an 100 g Kieselgel Si 60 (Merck 9385, 0,040 - 0,063 mm) in Chloroform-Methanol (98 : 2). Die reinen Fraktionen ($R_f$ = 0,38; Chloroform : Ethanol = 95 : 5) werden vereinigt und im Vakuum bei 40° eingedampft. Nach Umkristallisation des Rückstands aus 5 ml Essigsäureethylester erhält man N-(1-Desoxy-4,6-O-benzyliden-2-N-acetyl-muramyl)-staurosporin als beige Kristalle vom Smp. 246 - 248° (ab 238° Sintern), die noch 0,5 Mol Essigsäureethylester und 0,3 Mol Wasser enthalten; $[\alpha]_D^{20}$ = + 269,2 ± 2,1° (c = 0,483; Chloroform).

Beispiel 26:

Analog Beispiel 23 erhält man aus 602 mg (1,0 mMol) 4-O-Acetyl-6-O-stearoyl-2-N-acetyl-muraminsäure (1-α-Anomeres), die noch 0,17 Mol Wasser enthält, 195 mg (1,3 mMol) 1-Hydroxybenzotriazol, 268 mg (1,3 mMol) N,N′-Dicyclohexylcarbodiimid und 373 mg (0,8 mMol) Staurosporin in 20 ml absolutem N,N′-Dimethylformamid nach einer Reaktionszeit von insgesamt 22 Stunden (4 Stunden bei 0°, 18 Stunden bei Raumtemperatur), säulenchromatographischer Reinigung (150 g Kieselgel Si 60, Merck 9385, 0,040 - 0,063 mm) in Chloroform-Ethanol (95 : 5; 15 ml Fraktionen) und weiterer säulenchromatographischer Reinigung der Fraktionen 27 - 34 (100 g Kieselgel Si 60, Merck 9385, 0,040 - 0,063 mm) in Chloroform (ab Fraktion 600 in Chloroform-Methanol [98 : 2], jeweils 15 ml Fraktionen), sowie Umkristallisation der Fraktionen 624 - 639 aus 5 ml Essigsäureethylester N-(4-O-Acetyl-6-O-stearoyl-2-N-acetyl-muramyl)-staurosporin (1-α-Anomeres) als beige Kristalle vom Smp. 152,2 - 155,4° (ab 139° Sintern), die noch 0,45 Mol Wasser enthalten; $[\alpha]_D^{20}$ = + 143,5 ± 1,9° (c = 0,528; Methanol).

Das Ausgangsmaterial erhält man folgendermassen:

Stufe 26.1:

4,10 g (5,25 mMol) 1-α-O-Benzyl-4-O-acetyl-6-O-stearoyl-2-N-acetyl-muraminsäure-benzylester werden in 200 ml absolutem Methanol mit insgesamt 2,0 g 5%iger Palladiumkohle (Degussa E101N) unter Normaldruck bei Raumtemperatur 92 Stunden katalyrisch hydriert. Danach wird vom Katalysator abfiltriert und das Filtrat im Hochvakuum bei 30° zur Trockne eingedampft. Den Rückstand (farbloser Schaum) reinigt man durch Säulenchromatographie an 360 g Kieselgel Si 60 (Merck 9385, 0,040 - 0,063 mm) in Chloroform - Methanol (4 : 1; 20 ml Fraktionen). Die Fraktionen 120 - 390 werden vereinigt und im Hochvakuum bei 30° eingedampft. Der Rückstand (farbloser Schaum) wird in 200 ml Chloroform gelöst, die Lösung mit 50 ml 0,1-normaler Salzsäure versetzt und 5 Minuten bei Raumtemperatur gerührt. Nach Abtrennen der Chloroformphase wird diese mit 100 ml Wasser gewaschen, über Natriumsulfat getrocknet, filtriert und erneut eingedampft. Nach Umkristallisation des Rückstands aus 15 ml Ethanol erhält man 4-O-Acetyl-6-O-stearoyl-2-N-acetyl-muraminsäure (1-α-Anomeres) als farblose Kristalle vom Smp. 148,2 - 152,1° (ab 122° Sintern), die noch 0,17 Mol Wasser und 4,4 % Sulfatasche (von Säulenchromatographie) enthalten; $[\alpha]_D^{20}$ = + 43,1 ± 1,7° (c = 0,591; Chloroform : Methanol = 1 : 1). Die Substanz wird ohne weitere Reinigung verarbeitet.

Beispiel 27:

Analog Beispiel 23 erhält man aus 377 mg (0,97 mMol) 4,6-O-Diacetyl-2 -N-acetyl-muraminsäure (1-α-Anomeres), die noch 0,55 Mol Wasser enthält, 195 mg (1,3 mMol) 1-Hydroxybenzotriazol, 268 mg (1,3 mMol) N,N′-Dicyclohexylcarbodiimid und 373 mg (0,8 mMol) Staurosporin in 20 ml absolutem N,N′-Dimethylformamid nach einer Reaktionszeit von insgesamt 46 Stunden (4 Stunden 0°, 42 Stunden Raumtemperatur), säulenchromatographischer Reinigung (100 g Kieselgel Si 60, Merck 9385, 0,040 - 0,063 mm) in Chloroform (15 ml Fraktionen; ab Fraktion 200 in Chloroform-Methanol [98 : 2]) und Umkristallisation der Fraktionen 285 - 350 aus 10 ml Essigsäureethylester N-(4,6-O-Diacetyl-2-N-acetyl-muramyl)-staurosporin (1-α-Anomeres) als beige Kristalle vom Smp. 215 - 217° (ab 212° Sintern), die noch 1,61 Mol Wasser enthalten; $[\alpha]_D^{20}$ = + 177,6 ± 2,2° (c = 0,447; Methanol).

Das Ausgangsmaterial erhält man wie folgt:

Stufe 27.1:

Analog Beispiel 26, Stufe 26.1 erhält man aus 3,70 g (6,63 mMol) 1-α-O-Benzyl-4,6-O-diacetyl-2-N-acetyl-muraminsäure-benzylester durch katalytische Hydrierung unter Normaldruck bei Raumtemperatur in 200 ml Methanol mit insgesamt 1,5 g 5%iger Palladiumkohle (Degussa E 101 N; Hydrierdauer 70 Stunden) und säulenchromatographischer Reinigung an 320 g Kieselgel Si 60 (Merck 9385, 0,040 - 0,063 mm) in Methylenchlorid-Methanol-Wasser (70 : 30 : 5; 15 ml Fraktionen) nach Vereinigung und Eindampfen der Fraktionen 78 - 150 die rohe 4,6-O-Diacetyl-2-N-acetyl-muraminsäure als farblose Kristalle. Diese werden in 60 ml Wasser gelöst und die Lösung mit 15 ml einnormaler Salzsäure auf pH1 gestellt. Danach wird die wässrige Lösung mit Kochsalz gesättigt und zweimal mit je 150 ml Essigsäureethylester extrahiert. Die organischen Phasen werden vereinigt, über Natriumsulfat getrocknet und erneut eingedampft. Nach Kristallisation des Rückstands aus 35 ml Essigsäureethylester-n-Pentan (1 : 6) erhält man 4,6-O-Diacetyl-2-N-acetyl-muraminsäure (1-α-Anomeres) als farblose, hygroskopische Kristalle vom Smp. 85,8 - 90,8° (ab 78° Sintern), die noch 0,55 Mol Wasser enthalten; $[\alpha]_D^{20}$ = + 57,5 ± 2,2° (c = 0,449; Methanol).

Beispiel 28:

Analog Beispiel 23 erhält man aus 317 mg (0,48 mMol) 1-α,4-O-Diacetyl-6-O-stearoyl-2-N-acetyl-muraminsäure, die noch 0,78 Mol Wasser enthält, 98 mg (0,65 mMol) 1-Hydroxybenzotriazol, 134 mg (0,65 mMol) N,N′-Dicyclohexylcarbodiimid und 233 mg (0,5 mMol) Staurosporin in 10 ml absolutem N,N′-Dimethylformamid nach einer Reaktionszeit von insgesamt 46 Stunden (4 Stunden 0°, 42 Stunden Raumtemperatur), säulenchromatographischer Reinigung (50 g Kieselgel Si 60, Merck 9385, 0,040 - 0,063 mm) in Methylenchlorid-Methanol (99 : 1; 10 ml Fraktionen) und Kristallisation der Fraktionen 46 - 70 aus 10 ml Ethanol-Wasser (1 : 4) N-(1-α,4-O-Diacetyl-6-O-stearoyl-2-N-acetyl-muramyl)-staurosporin als beige Kristalle vom Smp. 146,4 - 148,2° (ab 142° Sintern), die noch 0,44 Mol Wasser enthalten; $[\alpha]_D^{20}$ = + 136,7 ± 1,7° (c = 0,580; Methanol).

Das Ausgangsmaterial erhält man folgendermassen:

Stufe 28.1:

Zu einer Lösung von 782 mg (1,29 mMol) 4-O-Acetyl-6-O-stearoyl-2-N-acetyl-muraminsäure (1-α-Anomeres), die noch 0,17 Mol Wasser enthält, in 20 ml absolutem Pyridin gibt man 204 mg (2,0 mMol; 0,189 ml) Essigsäureanhydrid und rührt 44 Stunden bei Raumtemperatur. Die so erhaltene gelbe Lösung wird im Hochvakuum bei 30° zur Trockne eingedampft. Den Rückstand (beiger Schaum) reinigt man durch Säulenchromatographie an 100 g Kieselgel Si 60 (Merck 9385, 0,040 - 0,063 mm) in Chloroform-Methanol (7 : 3; 15 ml Fraktionen). Die Fraktionen 19 - 26 werden vereinigt und erneut eingedampft. Den Rückstand löst man in 50 ml Wasser, säuert die so erhaltene Lösung mit einnormaler Salzsäure an (pH1) und extrahiert zweimal mit je 100 ml Essigsäureethylester. Die organischen Phasen werden vereinigt, über Natriumsulfat getrocknet und im Vakuum eingedampft. Der Rückstand (farbloser Schaum) kristallisiert aus 21 ml Diethylether-Petrolether (1 : 20). Man erhält 1-α,4-O-Diacetyl-6-O-stearoyl-2-N-acetyl-muraminsäure als farblose Kristalle vom Smp. 105,3 - 107,5°, die noch 0,78 Mol Wasser und etwas Petrolether enthalten; $[\alpha]_D^{20}$ = + 63,9 ± 1,9° (c = 0,532; Methanol).

Beispiel 29:

Analog Beispiel 23 erhält man aus 235 mg (0,56 mMol) 1-α,4,6-O-Triacetyl-2-N-acetyl-muraminsäure, die noch 0,14 Mol Wasser enthält, 109 mg (0,73 mMol) 1-Hydroxybenzotriazol, 150 mg (0,73 mMol) N,N'-Dicyclohexylcarbodiimid und 261 mg (0,56 mMol) Staurosporin in 10 ml N,N'-Dimethylformamid nach einer Reaktionszeit von insgesamt 46 Stunden (4 Stunden 0°, 42 Stunden Raumtemperatur), säulenchromatographischer Reinigung (30 g Kieselgel Si 60, Merck 9385, 0,040 - 0,063 mm) in Methylenchlorid - Methanol (99 : 1; 10 ml Fraktionen) und Aufschlemmung in Diethylether der Fraktionen 145 - 200 N-(1-α,4,6-O-Triacetyl-2-N-acetyl-muramyl)-staurosporin als beige Kristalle vom Smp. 199 - 204° (ab 182° Sintern), die noch 1,22 Mol Wasser und etwas Diethylether enthalten; $[\alpha]_D^{20}$ = + 144,7 ± 2,2° (c = 0,456; Methanol).

Das Ausgangsmaterial erhält man wie folgt:

Stufe 29.1:

Analog Beispiel 28, Stufe 28.1 erhält man aus 570 mg (1,47 mMol) 4,6-O-Diacetyl-2-N-acetyl-muraminsäure (1-α-Anomeres), die noch 0,55 Mol Wasser enthält, mit 308 mg (3,02 mMol; 0,286 ml) Essigsäureanhydrid in 20 ml absolutem Pyridin nach einer Reaktionszeit von 42 Stunden bei Raumtemperatur, säulenchromatographischer Reinigung des Rohprodukts an 100 g Kieselgel Si 60 (Merck 9385, 0,040 - 0,063 mm) in Chloroform-Methanol-Wasser (70 : 30 : 5; 10 ml Fraktionen) und Kristallisation der Fraktionen 43 - 105 aus 21 ml Methylenchlorid-Diethylether (1 : 20) 1-α,4,6-O-Triacetyl-2-N-acetyl-muraminsäure als farblose Kristalle vom Smp. 87,4 - 90,5° (ab 84° Sintern), die noch 0,14 Mol Wasser enthalten; $[\alpha]_D^{20}$ = + 102,4 ± 2,0° (c = 0,494; Methanol).

Beispiel 30:

Analog den in dieser Anmeldung beschriebenen Verfahren erhält man die folgenden Verbindungen:

a) N-(1-Desoxy-6-O-acetyl-2-N-acetyl-muramyl)-staurosporin, enthält 1,73 Mol Wasser, Smp. 225-227° (bei 218° Sintern; aus Essigsäureethylester: n-Pentan = 1 : 5), $[\alpha]_D^{20}$ = + 197,4 ± 1,8° (c = 0,549; Methanol),

b) N-(1-Desoxy-6-O-mesyl-2-N-acetyl-muramyl)-staurosporin, enthält 1,78 Mol Wasser, Smp. 231-233° (bei 222° Sintern; aus Essigsäureethylester), $[\alpha]_D^{20}$ = + 188,8 ± 2,1° (c = 0,472; Methanol),

c) N-(1-Desoxy-6-O-toluolsulfonyl-2-N-acetyl-muramyl)-staurosporin, enthält 0,83 Mol Wasser, Smp. 219-221° (aus Essigsäureethylester), $[\alpha]_D^{20}$ = + 192,1 ± 2,0° (c = 0,507; Chloroform: Methanol = 1 : 1),

d) N-(1-Desoxy-6-azido-2-N-acetyl-muramyl)-staurosporin, enthält 0,82 Mol Wasser, Smp. 291-293° (aus Essigsäureethylester), $[\alpha]_D^{20}$ = + 216,9 ± 2,1° (c = 0,474; Chloroform : Methanol = 1 : 1) und

e) N-(1-Desoxy-6-amino-2-N-acetyl-muramyl)-staurosporin-methansulfonat, enthält 3,90 Mol Wasser, Smp. 255-257° (bei 252° Sintern; aus Methanol: Essigsäureethylester = 3 : 25), $[\alpha]_D^{20}$ = + 175,0 ± 1,9° (c = 0,540; Methanol).

Beispiel 31:

Tabletten enthaltend 20 mg an Wirkstoff, z.B. eine der in den vorangehenden Beispielen beschriebenen Verbindungen der Formel I, werden in folgender Zusammensetzung in üblicher Weise hergestellt:

| Zusammensetzung: | |
|---|---|
| Wirkstoff | 20 mg |

| | |
|---|---|
| Weizenstärke | 60 mg |
| Milchzucker | 50 mg |
| Kolloidale Kieselsäure | 5 mg |
| Talk | 9 mg |
| Magnesiumstearat | 1 mg |
| | 145 mg |

Herstellung:

Der Wirkstoff wird mit einem Teil der Weizenstärke, mit Milchzucker und kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist.

Die plastische Masse wird durch ein Sieb von ca. 3 mm Maschenweite gedrückt, getrocknet und das erhaltene trockene Granulat nochmals durch ein Sieb getrieben. Darauf werden die restliche Weizenstärke, Talk und Magnesiumstearat zugemischt und die Mischung zu Tabletten von 145 mg Gewicht mit Bruchkerbe verpresst.

**Patentansprüche**

**1.** Staurosporinderivate der Formel I,

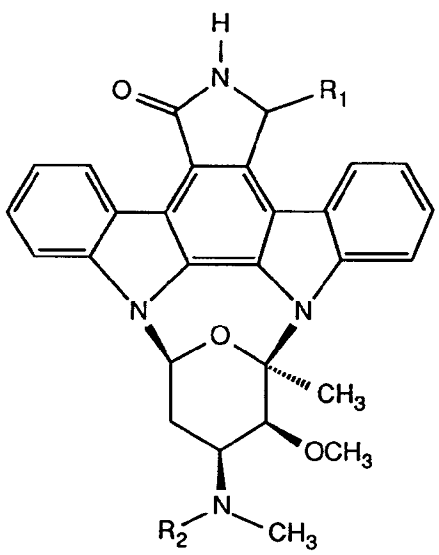

(I)

worin $R_1$ für Wasserstoff, Hydroxy, Niederalkoxy oder Oxo und $R_2$ für einen Rest der Formel II stehen,

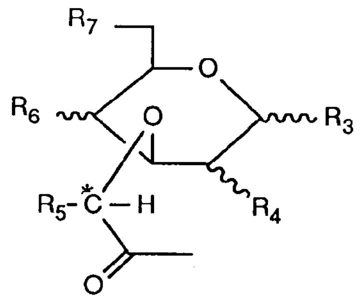

(II)

*(D) oder (L)-Konfiguration

worin sich die Konfiguration des Zuckerteils von D-Glucose, D-Galactose oder D-Mannose ableitet, und $R_3$ Wasserstoff, Hydroxy, Niederalkanoyloxy, Niederalkoxy oder jeweils unsubstituiertes oder im Phenylteil durch Halogen, Hydroxy, Trifluormethyl, Niederalkyl oder Niederalkoxy substituiertes Benzyloxy, Benzoyloxy oder Phenyloxy,

$R_4$ Hydroxy, Niederalkanoyloxy, Benzoyloxy, Benzyloxy, Amino, Niederalkylamino, Diniederalkylamino, Niederalkoxycarbonylamino, $C_2$-$C_{20}$-Alkanoylamino oder jeweils unsubstituiertes oder im Phenylteil durch Halogen, Hydroxy, Trifluormethyl, Niederalkyl oder Niederalkoxy substituiertes Benzoylamino, Benzyloxycarbonylamino oder Phenyloxycarbonylamino,

$R_5$ Wasserstoff oder Niederalkyl,

$R_6$ freies oder mit einer aliphatischen $C_2$-$C_{22}$-Carbonsäure verestertes Hydroxy, Niederalkoxycarbonyloxy, Niederalkylsulfonyloxy, freies oder mit einer aliphatischen $C_2$-$C_{22}$-Carbonsäure acyliertes Amino, Niederalkoxycarbonylamino, Azido oder jeweils unsubstituiertes oder im Phenylteil durch Halogen, Hydroxy, Trifluormethyl, Niederalkyl oder Niederalkoxy substituiertes Benzoyloxy, Benzyloxycarbonyloxy, Benzoylamino, Benzyloxycarbonylamino oder Phenylsulfonyloxy, und

$R_7$ freies oder mit einer aliphatischen $C_2$-$C_{22}$-Carbonsäure verestertes Hydroxy, Niederalkoxycarbonyloxy, Niederalkylsulfonyloxy, Azido, freies oder mit einer aliphatischen $C_2$-$C_{22}$-Carbonsäure acyliertes Amino, Niederalkylamino, Diniederalkylamino, Niederalkoxycarbonylamino, Carbamoylamino oder jeweils unsubstituiertes oder im Phenylteil durch Halogen, Hydroxy, Trifluormethyl, Niederalkyl, Niederalkoxy oder Niederalkoxycarbonyl substituiertes Benzoyloxy, Benzyloxycarbonyloxy, Phenylsulfonyloxy, Benzoylamino, Benzylamino oder Benzyloxycarbonylamino bedeuten, und Säureadditionssalze dieser Verbindungen mit mindestens einer salzbildenden Gruppe.

2. Verbindungen der Formel I nach Anspruch 1, worin $R_3$ Hydroxy, Niederalkanoyloxy, Niederalkoxy oder jeweils unsubstituiertes oder im Phenylteil durch Halogen, Hydroxy, Trifluormethyl, Niederalkyl oder Niederalkoxy substituiertes Benzyloxy, Benzoyloxy oder Phenyloxy und
$R_4$ Amino, Niederalkylamino, Diniederalkylamino, Niederalkoxycarbonylamino, $C_2$-$C_{20}$-Alkanoylamino oder jeweils unsubstituiertes oder im Phenylteil durch Halogen, Hydroxy, Trifluormethyl, Niederalkyl oder Niederalkoxy substituiertes Benzoylamino, Benzyloxycarbonylamino oder Phenyloxycarbonylamino bedeuten, und Säureadditionssalze dieser Verbindungen mit mindestens einer salzbildenden Gruppe.

3. Verbindungen der Formel I nach Anspruch 1 oder 2, worin $R_6$ freies oder mit einer unsubstituierten aliphatischen $C_2$-$C_{22}$-Carbonsäure verestertes Hydroxy, Niederalkoxycarbonyloxy, Niederalkylsulfonyloxy, freies oder mit einer unsubstituierten aliphatischen $C_2$-$C_{22}$-Carbonsäure acyliertes Amino, Niederalkoxycarbonylamino, Azido oder jeweils unsubstituiertes oder im Phenylteil durch Halogen, Hydroxy, Trifluormethyl, Niederalkyl oder Niederalkoxy substituiertes Benzoyloxy, Benzyloxycarbonyloxy, Benzoylamino, Benzyloxycarbonylamino oder Phenylsulfonyloxy, und
$R_7$ freies oder mit einer unsubstituierten aliphatischen $C_2$-$C_{22}$-Carbonsäure verestertes Hydroxy, Niederalkoxycarbonyloxy, Niederalkylsulfonyloxy, Azido, freies oder mit einer unsubstituierten aliphatischen $C_2$-$C_{22}$-Carbonsäure acyliertes Amino, Niederalkylamino, Diniederalkylamino, Niederalkoxycarbonylamino, Carbamoylamino oder jeweils unsubstituiertes oder im Phenylteil durch Halogen, Hydroxy, Trifluormethyl, Niederalkyl, Niederalkoxy oder Niederalkoxycarbonyl substituiertes Benzoyloxy, Benzyloxycarbonyloxy, Phenylsulfonyloxy, Benzoylamino, Benzylamino oder Benzyloxycarbonylamino bedeuten, und Säureadditionssalze dieser Verbindungen mit mindestens einer salzbildenden Gruppe.

4. Verbindungen der Formel I nach Anspruch 1, worin $R_1$ Wasserstoff und $R_6$ freies oder mit einer unverzweigten $C_2$-$C_{22}$-Alkansäure oder einer unverzweigten $C_2$-$C_{22}$-Alkensäure verestertes Hydroxy,

Niederalkoxycarbonyloxy, Niederalkylsulfonyloxy, freies oder mit einer unverzweigten $C_2$-$C_{22}$-Alkansäure oder einer unverzweigten $C_2$-$C_{22}$-Alkensäure acyliertes Amino, Niederalkoxycarbonylamino, Azido oder jeweils unsubstituiertes oder im Phenylteil durch Halogen, Hydroxy, Trifluormethyl, Niederalkyl oder Niederalkoxy substituiertes Benzoyloxy, Benzyloxycarbonyloxy, Benzoylamino, Benzyloxycarbonylamino oder Phenylsulfonyloxy, und

$R_7$ freies oder mit einer unverzweigten $C_2$-$C_{22}$-Alkansäure oder einer unverzweigten $C_2$-$C_{22}$-Alkensäure verestertes Hydroxy, Niederalkoxycarbonyloxy, Niederalkylsulfonyloxy, Azido, freies oder mit einer unverzweigten $C_2$-$C_{22}$-Alkansäure oder einer unverzweigten $C_2$-$C_{22}$-Alkensäure acyliertes Amino, Niederalkylamino, Diniederalkylamino, Niederalkoxycarbonylamino, Carbamoylamino oder jeweils unsubstituiertes oder im Phenylteil durch Halogen, Hydroxy, Trifluormethyl, Niederalkyl, Niederalkoxy oder Niederalkoxycarbonyl substituiertes Benzoyloxy, Benzyloxycarbonyloxy, Phenylsulfonyloxy, Benzoylamino, Benzylamino oder Benzyloxycarbonylamino bedeuten, und Säureadditionssalze dieser Verbindungen mit mindestens einer salzbildenden Gruppe.

5. Verbindungen der Formel I nach einem der Ansprüche 1-4, worin sich die Konfiguration des Zuckerteils von D-Glucose ableitet, und Säureadditionssalze von solchen Verbindungen mit mindestens einer salzbildenden Gruppe.

6. Verbindungen der Formel I nach einem der Ansprüche 1-5, die am C*-Atom die (D)-Konfiguration aufweisen, und Säureadditionssalze von solchen Verbindungen mit mindestens einer salzbildenden Gruppe.

7. Verbindungen der Formel I nach Anspruch 1, worin sich die Konfiguration des Zuckerteils von D-Glucose ableitet, $R_1$ Wasserstoff und $R_2$ einen Rest der Formel II bedeuten, worin $R_3$ Wasserstoff, Hydroxy, Benzyloxy, Niederalkoxy oder Niederalkanoyloxy, $R_4$ Niederalkanoylamino, $R_5$ Niederalkyl oder Wasserstoff, $R_6$ Hydroxy oder Niederalkanoyloxy und $R_7$ Hydroxy, Niederalkylsulfonyloxy, Azido, Amino oder Alkanoyloxy mit bis zu 24 C-Atomen bedeuten, und Säureadditionssalze von solchen Verbindungen mit mindestens einer salzbildenden Gruppe.

8. Verbindungen der Formel I nach Anspruch 1, worin sich die Konfiguration des Zuckerteils von D-Glucose ableitet, $R_1$ Wasserstoff und $R_2$ einen Rest der Formel II bedeuten, worin $R_3$ Wasserstoff, Hydroxy, Benzyloxy, Niederalkoxy oder Niederalkanoyloxy, $R_4$ $C_1$-$C_4$-Alkanoylamino, $R_5$ $C_1$-$C_4$-Alkyl oder Wasserstoff, $R_6$ Hydroxy oder Niederalkanoyloxy und $R_7$ Hydroxy, Methylsulfonyloxy, Azido, Amino oder Alkanoyloxy mit bis zu 24 C-Atomen bedeuten, und Säureadditionssalze von solchen Verbindungen mit mindestens einer salzbildenden Gruppe.

9. Verbindungen der Formel I nach Anspruch 1, worin sich die Konfiguration des Zuckerteils von D-Glucose ableitet, $R_1$ Wasserstoff und $R_2$ einen Rest der Formel II bedeuten, worin $R_3$ Hydroxy, Benzyloxy oder Niederalkoxy, $R_4$ Niederalkanoylamino, $R_5$ Niederalkyl oder Wasserstoff, $R_6$ Hydroxy und $R_7$ Hydroxy, Niederalkylsulfonyloxy, Azido oder Amino bedeuten, und Säureadditionssalze von solchen Verbindungen mit mindestens einer salzbildenden Gruppe.

10. Eine Verbindung der Formel I oder ein Säureadditionssalz einer solchen Verbindung mit mindestens einer salzbildenden Gruppe nach Anspruch I ausgewählt aus
N-(1-α-O-Benzyl-2-N-acetyl-muramyl)-staurosporin,
N-(2-N-Acetyl-muramyl)-staurosporin,
N-(6-O-Mesyl-1-α-O-benzyl-2-N-acetyl-muramyl)-staurosporin,
N-(6-Azido- 1-α-O-benzyl-2-N-acetyl-6-desoxy-muramyl)-staurosporin,
N-(6-Amino-1-α-O-benzyl-2-N-acetyl-6-desoxy-muramyl)-staurosporin oder einem pharmazeutisch verwendbaren Säureadditionssalz davon,
N-(6-Amino-6-desoxy-2-N-acetyl-muramyl)-staurosporin oder einem pharmazeutisch verwendbaren Säureadditionssalz davon,
N-(6-O-Mesyl-2-N-acetyl-muramyl)-staurosporin,
N-(2-N-Acetyl-desmethylmuramyl)-staurosporin,
N-(1-α-O-Benzyl-2-N-acetyl-homomuramyl)-staurosporin,
N-(1-α-O-Benzyl-2-N-acetyl-L-homomuramyl)-staurosporin,
dem 1-α-Anomeren von N-(2-N-Acetyl-L-homomuramyl)-staurosporin,
N-(1-α-O-Benzyl-4,6-O-diacetyl-2-N-acetyl-muramyl)-staurosporin,
N-(1-α-O-Benzyl-4-O-acetyl-6-O-stearoyl-2-N-acetyl-muramyl)-staurosporin,
N-(1-Desoxy-2-N-acetyl-muramyl)-staurosporin,

N-(4-O-Acetyl-6-O-stearoyl-2-N-acetyl-muramyl)-staurosporin,
dem 1-α-Anomeren von N-(4,6-O-Diacetyl-2-N-acetyl-muramyl)-staurosporin,
N-(1-α,4-O-Diacetyl-6-O-stearoyl-2-N-acetyl-muramyl)-staurosporin,
N-(1-α,4,6-O-Triacetyl-2-N-acetyl-muramyl)-staurosporin,
N-(1-Desoxy-6-O-acetyl-2-N-acetyl-muramyl)-staurosporin,
N-(1-Desoxy-6-O-mesyl-2-N-acetyl-muramyl)-staurosporin,
N-(1-Desoxy-6-O-toluolsulfonyl-2-N-acetyl-muramyl)-staurosporin,
N-(1-Desoxy-6-azido-2-N-acetyl-muramyl)-staurosporin und
N-(1-Desoxy-6-amino-2-N-acetyl-muramyl)-staurosporin oder einem pharmazeutisch verwendbaren Säureadditionssalz davon.

**11.** Eine Verbindung der Formel I nach einem der Ansprüche 1-10 oder ein pharmazeutisch verwendbares Säureadditionssalz einer solchen Verbindung mit mindestens einer salzbildenden Gruppe zur Anwendung in einem Verfahren zur therapeutischen Behandlung des menschlichen oder tierischen Körpers.

**12.** Pharmazeutische Präparate, die eine Verbindung der Formel I nach einem der Ansprüche 1-10 oder ein pharmazeutisch verwendbares Säureadditionssalz einer solchen Verbindung mit mindestens einer salzbildenden Gruppe zusammen mit pharmazeutischem Trägermaterial enthalten.

**13.** Verwendung einer Verbindung der Formel I nach einem der Ansprüche 1-10 oder eines pharmazeutisch verwendbaren Säureadditionssalzes einer solchen Verbindung mit mindestens einer salzbildenden Gruppe zur Herstellung von pharmazeutischen Präparaten, die für die Anwendung zur Behandlung von Krankheiten bestimmt sind, welche auf eine Hemmung der Proteinkinase C ansprechen.

**14.** Verfahren zur Herstellung einer Verbindung der Formel I nach Anspruch 1 oder eines Säureadditionssalzes einer solchen Verbindung mit mindestens einer salzbildenden Gruppe, dadurch gekennzeichnet, dass man ein Amin der Formel V,

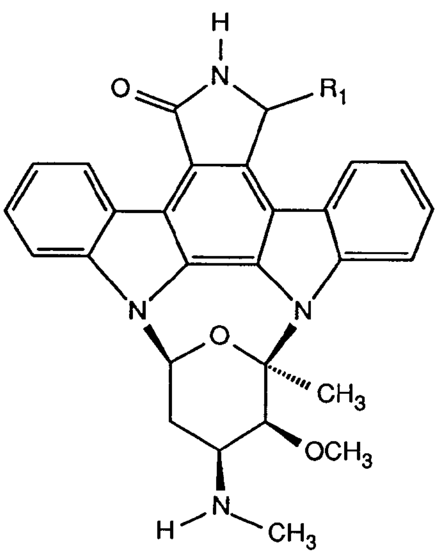

(V)

worin $R_1$ die obengenannte Bedeutung hat mit der Massgabe, dass eine durch $R_1$ repräsentierte Hydroxygruppe erforderlichenfalls durch eine leicht abspaltbare Hydroxyschutzgruppe geschützt ist, mit einer Carbonsäure der Formel VI,

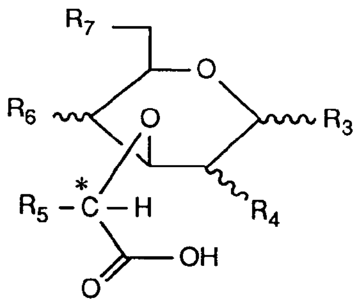

(VI)

*(D) oder (L)-Konfiguration

worin die Substituenten die obengenannten Bedeutungen haben mit der Massgabe, dass in einer Verbindung der Formel VI vorhandene freie funktionelle Gruppen mit Ausnahme der an der Reaktion teilnehmenden Carboxylgruppe erforderlichenfalls durch leicht abspaltbare Schutzgruppen geschützt sind, oder einer entsprechenden Carbonsäure in der Furanoseform, oder einem reaktionsfähigen Carbonsäurederivat davon acyliert und Schutzgruppen, welche im gewünschten Endprodukt der Formel I nicht vorhanden sind, abspaltet und, wenn erwünscht, eine erhaltene Verbindung der Formel I in eine andere Verbindung der Formel I überführt oder eine erhaltene Verbindung der Formel I mit mindestens einer salzbildenden Gruppe in ihr Säureadditionssalz überführt, oder ein erhaltenes Säureadditionssalz einer Verbindung der Formel I in die freie Verbindung umwandelt, und/oder ein erhaltenes Isomerengemisch auftrennt.

Europäisches Patentamt

# EUROPÄISCHER RECHERCHENBERICHT

Nummer der Anmeldung

EP 92 81 0831

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
| A | WO-A-9 109 034 (SCHERING CORPORATION)<br>* Zusammenfassung *<br>--- | 1,12,13 | C07H15/26<br>A61K31/55 |
| A | EP-A-0 383 919 (KYOWA HAKKO KOGYO CO., LTD.)<br>* das ganze Dokument *<br>--- | 1,12,13 | |
| A | EP-A-0 296 110 (CIBA-GEIGY AG)<br>* das ganze Dokument *<br>----- | 1,12,13 | |
| | | | RECHERCHIERTE SACHGEBIETE (Int. Cl.5 ) |
| | | | C07H<br>A61K |

Der vorliegende Recherchenbericht wurde für alle Patentansprüche erstellt

| Recherchenort | Abschlußdatum der Recherche | Prüfer |
|---|---|---|
| DEN HAAG | 11 JANUAR 1993 | SCOTT J.R. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)